(19) Europäisches Patentamt
European Patent Office
Office européen des brevets

(11) EP 2 345 431 A2

(12) EUROPÄISCHE PATENTANMELDUNG

(43) Veröffentlichungstag:
**20.07.2011 Patentblatt 2011/29**

(51) Int Cl.:
***A61L 15/60*** *(2006.01)* ***C07C 67/08*** *(2006.01)*
***C08G 65/332*** *(2006.01)*

(21) Anmeldenummer: **10177026.1**

(22) Anmeldetag: **06.06.2003**

(84) Benannte Vertragsstaaten:
**AT BE BG CH CY CZ DE DK EE ES FI FR GB GR HU IE IT LI LU MC NL PT RO SE SI SK TR**

(30) Priorität: **11.06.2002 DE 10225943**

(62) Dokumentnummer(n) der früheren Anmeldung(en) nach Art. 76 EPÜ:
**03757035.5 / 1 516 010**

(27) Früher eingereichte Anmeldung:
**06.06.2003 EP 03757035**

(71) Anmelder: **BASF SE**
**67056 Ludwigshafen (DE)**

(72) Erfinder:
- **Jaworek, Thomas**
**67169, Kallstadt (DE)**
- **Daniel, Thomas**
**67165, Waldsee (DE)**
- **Wolf, Lothar**
**02991, Leippe-Torno (DE)**
- **Königer, Rainer**
**85375, Neufahrn (DE)**
- **Schwalm, Reinhold**
**67157, Wachenheim (DE)**
- **Hartmann, Gabriele**
**68766, Hockenheim (DE)**
- **Wickel, Stefan**
**67281, Bissersheim (DE)**

Bemerkungen:
Diese Anmeldung ist am 16-09-2010 als Teilanmeldung zu der unter INID-Code 62 erwähnten Anmeldung eingereicht worden.

# (54) Verfahren zur Herstellung von Estern von Polyalkoholen

(57) Verfahren zur Veresterung ungesättigter Säuren mit Polyalkoholen und Verwendung der so erhältlichen Reaktionsgemische.

EP 2 345 431 A2

## Beschreibung

Die vorliegende Erfindung betrifft ein vereinfachtes Verfahren zur Veresterung ungesättigter Säuren mit Polyalkoholen und Verwendung der so erhältlichen Reaktionsgemische.

Quellbare Hydrogel-bildende Polymerisate, sogenannte Superabsorber (Super-Absorbing Polymers, SAP), sind aus dem Stand der Technik bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. Superabsorber weren außerdem in anderen Gebieten der Technik eingesetzt, bei denen Flüssigkeiten, insbesondere Wasser oder wässrige Lösungen absorbiert werden. Diese Gebiete sind z.B. Lagerung, Verpackung, Transport (Verpackungsmaterial wassersensitiver Artikel, wie z.B. Blumentransport, Schock Protection); Lebensmittelsektor (Transport von Fisch, Frischfleisch; Absorption von Wasser, Blut in Frischfisch/-Fleisch-Verpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/ Öl-Emulsionen; Textil (Handschuhe, Sportbekleidung, Feuchtigkeitsregulation in Textilien, Schuheinlagen); chem.-techn. Anwendungen (Katalysator für org. Reaktionen, Immobilisierung großer funktioneller Moleküle (Enzyme), Adhesiv für Agglomerationen, Wärmespeicher, Filtrationshilfsmittel, hydrophile Komponente in Polymerlaminaten, Dispergiermittel, Verflüssiger); Bau- und Konstruktionswesen, Installation (Pulverspritzguss, lehmbasierende Putze, Vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freitsetzung von Wirkstoffen an Pflanzen); im Feuerschutz (Funkenflug) (Abdecken von Häusern bzw. Bedecken von Hauswänden mit SAP Gel, da das Wasser eine sehr hohe Wärmekapazität hat, kann ein Entflammen verhindert werden; Versprühen von SAP Gel bei Bränden wie z.B. Waldbränden); Coextrusionsmittel in thermoplastischen Polymeren (Hydrophilierung von Mehrschichtfolien) ; Herstellung von Folien und thermoplastischen Formkörpern, die Wasser absorbieren können (z.B. Regen- und Tauwasser speichernde Folien für die Landwirtschaft); SAP haltige Folien zum Frischhalten von Obst und Gemüse, die in feuchte Folien verpackt werden können; der SAP speichert vom Obst und Gemüse abgegebenes Wasser ohne Bildung von Kondensationstropfen und gibt das Wasser teilweise an das Obst und Gemüse wieder ab, so daß weder Faulen noch Verwelken einritt; SAP-Polystyrol Coextrudate z.B. für Lebensmittelverpackungen wie Fleisch, Fisch, Geflügel, Obst und Gemüse); Trägersubstanz in Wirkstoffformulierungen (Pharma, Pflanzenschutz). In den Hygieneartikeln befinden sich die Superabsorber in aller Regel im sog. absorbent core, der als weitere Materialien unter anderem Fasern (Cellulosefasern) umfasst, die als eine Art Flüssigkeitsreservoir die spontan beaufschlagten Flüssigkeitsmengen zwischenspeichern und eine gute Kanalisation der Körperflüssigkeiten im absorbent core hin zum Superabsorber gewährleisten sollen.

Der aktuelle Trend im Windelaufbau besteht darin, dünnere Konstruktionen mit reduziertem Cellulosefaseranteil und erhöhtem Hydrogelanteil herzustellen. Mit dem Trend zu immer dünner werdenden Windelkonstruktionen hat sich das Anforderungsprofil an die wasserquellbaren hydrophilen Polymeren über die Jahre deutlich verändert. Während zu Beginn der Entwicklung hochsaugfähiger Hydrogele zunächst allein das sehr hohe Quellvermögen in Vordergrund stand, hat sich später gezeigt, dass auch die Fähigkeit des Superabsorbers zur Flüssigkeitsweiterleitung und -verteilung von entscheidender Bedeutung ist. Es hat sich gezeigt, dass herkömmliche Superabsorber an der Oberfläche bei Benetzung mit Flüssigkeit stark anquellen, so dass der Flüssigkeitstransport ins Partikelinnere stark erschwert oder ganz unterbunden wird. Diese Eigenart des Superabsorbers wird auch als "Gelblocking" bezeichnet. Aufgrund der höheren Beladung des Hygieneartikels (Polymer pro Flächeneinheit) darf das Polymer im gequollenen Zustand keine Sperrschicht für nachfolgende Flüssigkeit bilden. Weist das Produkt gute Transporteigenschaften auf, so kann eine optimale Ausnutzung des gesamten Hygieneartikels gewährleistet werden. Das Phänomen des Gelblockings wird somit unterbunden, das im Extremfall zum Austritt der Flüssigkeit, der sog. Leakage des Hygieneartikels führt. Die Flüssigkeitsweiterleitung und -verteilung ist also bei der anfänglichen Absorption von Körperflüssigkeiten von entscheidender Bedeutung.

Gute Transporteigenschaften besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Vernetzung erreicht, wodurch allerdings die Retention des Produktes verringert wird. Eine elegante Methode zur Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein.

Hydrophile, hochquellfähige Hydrogele sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wäßrigen Flüssigkeiten quellbare Naturprodukte, wie beispielsweise Guarderivate. Solche Hydrogele werden als wäßrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Zur Verbesserung der Anwendungseigenschaften, wie z.B. Rewet in der Windel und AUL, werden hydrophile, hochquellfähige Hydrogele im allgemeinen oberflächen- oder gelnachvernetzt. Diese Nachvernetzung ist dem Fachmann an sich bekannt und erfolgt bevorzugt in wäßriger Gelphase oder als Oberflächennachvernetzung der gemahlenen und abgesiebten Polymerpartikel.

Aus WO 93/21237 sind (Meth)acrylate von alkoxylierten mehrwertigen $C_2$ - $C_{10}$-Kohlenwasserstoffen als Vernetzer bekannt. Diese können als Gemische mit Nebenprodukten aus dem Herstellungsprozeß eingesetzt werden.

Nachteilig an diesen Verbindungen ist, daß zur zumindest teilweisen Abtrennung von Einsatzstoffen und Nebenprodukten - die in der genannten Schrift eingesetzten Vernetzer weisen einen Gehalt an Acrylsäure von weniger als 0,1 Gew% auf - aufwendige Reinigungsoperationen erforderlich sind.

Die Herstellung solcher höheren (Meth)acrylsäureester durch säurekatalysierte Veresterung von (Meth)acrylsäure mit den entsprechenden Alkoholen in Gegenwart eines Inhibitors/Inhibitorsystems sowie gegebenenfalls eines Lösungsmittels, wie z.B. Benzol, Toluol, Cyclohexan, ist allgemein bekannt.

Da bekanntlich der Bildung des Esters aus (Meth)acrylsäure und Alkohol eine Gleichgewichtsreaktion zugrunde liegt, wird, um wirtschaftliche Umsätze zu erzielen, in der Regel ein Einsatzstoff im Überschuß eingesetzt und/oder das gebildete Veresterungswasser und/oder der Zielester aus dem Gleichgewicht entfernt.

Daher wird bei der Herstellung der höheren (Meth)acrylsäureester in der Regel das Reaktionswasser entfernt und meist ein Überschuß an (Meth)acrylsäure eingesetzt.

US 4 187 383 beschreibt ein Veresterungsverfahren von (Meth)acrylsäure mit organischen Polyolen bei einer Reaktionstemperatur von 20 bis 80 °C mit einem Äquivalentüberschuß von 2 bis 3 : 1.

Nachteilig an diesem Verfahren ist, daß durch die niedrige Reaktionstemperatur die Reaktionszeiten bis zu 35 Stunden betragen und der Überschuß Säure im Reaktionsgemisch durch eine Neutralisation mit anschließender Phasentrennung entfernt wird.

WO 2001/14438 (Derwent-Abstract Nr. 2001-191644/19) und WO 2001/10920 (Chemical Abstracts 134: 163502) beschreiben Verfahren zur Veresterung von (Meth)acrylsäure mit Polyalkylenglykolmonoalkylethern im Verhältnis 3:1 - 50:1 in Gegenwart von Säuren und Polymerisationsinhibitoren und, nach Desaktivierung des sauren Katalysators, Copolymerisation des Rückstandes aus (Meth)acrylsäureester und (Meth)acrylsäure bei pH 1,5 - 3,5, sowie dessen Verwendung als Zementadditiv.

Nachteilig an diesen Verfahren ist, daß es auf Polyalkylenglykolmonoalkylether beschränkt ist, daß der Katalysator desaktiviert werden muß und daß derartige Copolymerisate nicht als Vernetzer für Hydrogele eingesetzt werden können, da sie lediglich eine Funktionalität aufweisen.

Es bestand die Aufgabe, das Herstellungsverfahren für Substanzen, die als Radikalvernetzer für Superabsorber verwendbar sind, zu vereinfachen.

Die Aufgabe wird gelöst durch ein Verfahren zur Herstellung eines Esters F eines Polyalkohols A mit mindestens einer ethylenisch ungesättigten Carbonsäure B, umfassend die Schritte

a) Umsetzung eines Polyalkohols A mit mindestens einer ethylenisch ungesättigten Carbonsäure B in Anwesenheit mindestens eines Veresterungskatalysators C und mindestens eines Polymerisationsinhibitors D sowie gegebenenfalls eines mit Wasser ein Azeotrop bildenden Lösungsmittels E unter Bildung eines Esters F,
b) gegebenenfalls Entfernen zumindest eines Teils des in a) entstehenden Wassers aus dem Reaktionsgemisch, wobei b) während und/ oder nach a) erfolgen kann,
f) gegebenenfalls Neutralisation des Reaktionsgemischs,
h) falls ein Lösungsmittel E eingesetzt wurde gegebenenfalls Entfernen dieses Lösungsmittels durch Destillation und/oder
i) Strippen mit einem unter den Reaktionsbedingungen inerten Gas, wobei

- der Polyalkohol A mindestens zwei Hydroxyfunktionen aufweist,

- der molare Überschuß der ethylenisch ungesättigten Carbonsäure B zum Polyalkohol A je zu veresternder Hydroxygruppe in A mindestens 1,05:1 beträgt und
- die in dem nach dem letzten Schritt erhaltenen Reaktionsgemisch enthaltene, gegebenenfalls neutralisierte Carbonsäure B im wesentlichen im Reaktionsgemisch verbleibt.

Der molare Überschuß von B zu A beträgt (je zu veresternder Hydroxygruppe im Polyalkohol A) mindestens 1,05 : 1, bevorzugt mindestens 1,1:1, besonders bevorzugt mindestens 1,25:1, ganz besonders bevorzugt mindestens 1,5:1 und insbesondere mindestens 2,5:1.

In einer bevorzugten Ausführungsform wird B in einem Überschuß von beispielsweise größer als 5:1, bevorzugt größer als 10:1, besonders bevorzugt größer als 20:1, ganz besonders bevorzugt größer 50:1, insbesondere größer 75: 1 und speziell größer als 100:1 eingesetzt.

Die so erhältlichen Veresterungsprodukte können im wesentlichen ohne weitere Reinigung, besonders ohne wesentliche Abtrennung des Überschusses an Carbonsäure B und des Gehalts an Veresterungskatalysator C, als Radikalvernetzer in Hydrogelen eingesetzt werden.

Unter Vernetzung wird in dieser Schrift wenn nicht anders erwähnt die Radikalvernetzung (Gelvernetzung, Innenvernetzung, Quervernetzung von linearem oder schwach vernetzten Polymer) als verstanden. Diese Vernetzung kann über radikalische oder kationische Polymerisationsmechanismen oder andere, beispielsweise Michael-Addition, Ver- oder Umesterungsmechanismen erfolgen, bevorzugt durch radikalische Polymerisation.

Wässrige Flüssigkeiten absorbierende Hydrogel-formende Polymere sind bevorzugt solche mit einer Absorption von destilliertem Wasser von mindestens dem Eigengewicht, bevorzugt dem 10-fachem Eigengewicht, diese Absorption wird bevorzugt auch unter einem Druck von 0,7 psi erreicht.

Erfindungsgemäß einsetzbare Polyalkohole A sind Verbindungen, die mindestens zwei Hydroxyfunktionen (-OH) aufweisen, bevorzugt mindestens drei, besonders bevorzugt drei bis zehn, ganz besonders bevorzugt drei bis sechs und insbesondere drei bis vier.

Die Polyalkohole können aliphatisch, cycloaliphatisch oder aromatisch sein, bevorzugt aliphatisch oder cycloaliphatisch und ganz besonders bevorzugt aliphatisch, geradkettig oder verzweigt und gegebenenfalls substituiert mit funktionellen Gruppen.

In der Regel weisen die Polyalkohole zwei bis 50 Kohlenstoffatome auf und bevorzugt drei bis 40.

Das Molgewicht der einsetzbaren Polyalkohole beträgt in der Regel, wenn nicht anders angegeben, unter 5000 g/mol, bevorzugt unter 2500 g/mol, besonders bevorzugt unter 1500 g/mol, ganz besonders bevorzugt unter 1000 g/mol und insbesondere unter 800 g/ mol.

Bevorzugte Polyalkohole A sind Polyole, funktionalisierte Polyole, alkoxylierte Polyole, Zuckeralkohole, teilweise alkoxylierte Zuckeralkohole, Polyetherole, Polyesterole, zumindest teilweise alkoxylierte Polyesterole und zumindest teilweise verseifte, alkoxylierte Polyesterole.

Beispiele für Polyole sind Trimethylolbutan, Trimethylolpropan, Trimethylolethan, Neopentylglykol, Hydroxypivalinsäureneopentylglykolester, Pentaerythrit, Glycerin, 1,2-Ethylenglykol, 1,2-Propylenglykol, 2-Ethyl-1,3-Propandiol, 2-Methyl-1,3-Propandiol, Hydrochinon, Bisphenol A, Bisphenol F, Bisphenol B, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, But-2-en-1,4-diol und But-2-in-1,4-diol.

Die Polyole können auch noch zusätzliche Funktionalitäten tragen wie z.B. Etherfunktionen (-O-), Carboxylfunktionen (-COOH) oder $C_1$-$C_4$-Alkyloxycarbonylfunktionen (Estergruppen), wobei $C_1$-$C_4$-Alkyl in dieser Schrift Methyl, Ethyl, *iso*-Propyl, n-Propyl, n-Butyl, *iso*-Butyl, *sek*-Butyl oder *tert*-Butyl bedeutet.

Beispiele für solche funktionalisierten Polyole sind Ditrimethylolpropan, Dipentaerythrit, Dimethylolpropionsäure, Dimethylolbuttersäure, Trimethylolessigsäure, Hydroxypivalinsäure und die 2-Hydroxyethyl- oder $C_1$-$C_4$-Alkylester dieser genannten Säuren. Bevorzugte Polyole sind solche der Formel (I):

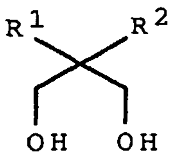

(I)

Darin bedeuten

$R^1$, $R^2$ unabhängig voneinander Wasserstoff, $C_1$ - $C_{10}$-Alkyl, bevorzugt $C_1$ - $C_4$-Alkyl, $C_1$ - $C_{10}$-Hydroxyalkyl, bevorzugt Hydroxy-$C_1$ - $C_4$-Alkyl, Carboxyl oder $C_1$ - $C_4$-Alkyloxy- carbonyl, bevorzugt Wasserstoff, Hydroxymethyl und

$C_1$ - $C_4$-Alkyl und besonders bevorzugt Hydroxymethyl und $C_1$ - $C_4$-Alkyl.

Dabei können die Alkylreste jeweils geradkettig oder verzweigt sein.

Beispiele für $R^1$ und $R^2$ sind Wasserstoff, Methyl, Ethyl, *iso*-Propyl, n-Propyl, n-Butyl, *iso*-Butyl, *sek*-Butyl, *tert*-Butyl, n-Pentyl, n-Hexyl, n-Heptyl, n-Octyl, n-Decyl, Hydroxymethyl, Carboxyl, Methoxycarbonyl, Ethoxycarbonyl oder n-Butoxycarbonyl, bevorzugt Wasserstoff, Hydroxymethyl, Methyl und Ethyl, besonders bevorzugt Hydroxymethyl, Methyl und Ethyl.

Besonders bevorzugte mehrwertige Alkohole der Formel (I) sind Trimethylolbutan, Trimethylolpropan, Trimethylolethan, Neopentylglykol, Pentaerythrit, 2-Ethyl-1,3-Propandiol, 2-Methyl-1,3-Propandiol, 1,3-Propandiol, Dimethylolpropionsäure, Dimethylolpropionsäuremethylester, Dimethylolpropionsäureethylester, Dimethylolbuttersäure, Dimethylolbuttersäuremethylester oder Dimethylolbuttersäureethylester, bevorzugt sind Neopentylglykol, Trimethylolpropan, Pentaerythrit und Dimethylolpropionsäure, ganz besonders bevorzugt sind Neopentylglykol, Trimethylolpropan und Pentaerythrit und insbesondere Trimethylolpropan und Pentaerythrit.

Beispiele für Zuckeralkohole sind Sorbit, Mannit, Maltit, Isomalt, Diglycerol, Threit, Erythrit, Adonit (Ribit), Arabit (Lyxit), Xylit und Dulcit (Galactit).

Beispiele für Polyetherole sind Poly-THF mit einer Molmasse zwischen 162 und 2000, bevorzugt zwischen 162 und 1458, besonders bevorzugt zwischen 162 und 1098, ganz besonders bevorzugt zwischen 162 und 738 und insbesondere zwischen 162 und 378, Poly-1,3-propandiol und Poly-1,2-propandiol mit einer Molmasse zwischen 134 und 1178, bevorzugt zwischen 134 und 888, besonders bevorzugt zwischen 134 und 598 und ganz besonders bevorzugt zwischen 134 und 308, Polyethylenglykol mit einer Molmasse zwischen 106 und 898, bevorzugt zwischen 106 und 458, besonders bevorzugt von 106 bis 400, ganz besonders bevorzugt zwischen 106 und 235 und insbesondere Diethylenglykol, Triethylenglykol und Tetraethylenglykol.

Als Polyesterole kommen z.B. solche in Betracht, wie sie durch Veresterung von Polycarbonsäuren, vorzugsweise Dicarbonsäuren, mit den oben genannten Polyolen hergestellt werden können.

Die Ausgangsstoffe für solche Polyesterole sind dem Fachmann bekannt. Bevorzugt können als Polycarbonsäuren Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, deren Isomere und Hydrierungsprodukte sowie veresterbare Derivate, wie Anhydride oder Dialkylester, beispielsweise $C_1$-$C_4$-Alkylester, bevorzugt Methyl-, Ethyl- oder n-Butylester, der genannten Säuren eingesetzt werden.

Als Hydroxygruppen tragende Carbonsäuren oder Lactone kommen 4-Hydroxybenzoesäure, 6-Hydroxy-2-naphthalinsäure, Pivalolacton oder ε-Caprolacton in Betracht. Als Polyole kommen die oben genannten mehrfunktionellen Alkohole, vorzugsweise Neopentylglykol, Trimethylolpropan, Trimethylolethan, Pentaerythrit, Dimethylolpropionsäure oder Dimethylolbuttersäure in Betracht.

Bevorzugte Beispiele für derartige Polyesterole sind solche der Formel (IIIa-c),

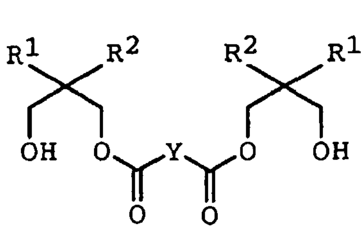

(IIIa)

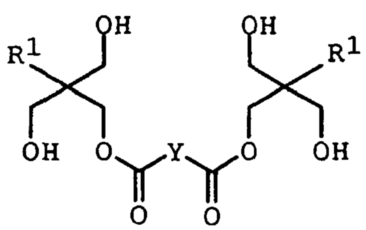

(IIIb)

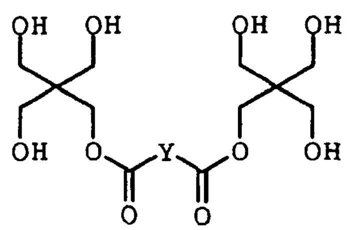

(IIIc)

worin

$R^1$, $R^2$ die oben genannten Bedeutungen haben und

Y eine geradkettige oder verzweigte, gegebenenfalls substituierte Alkylengruppe mit 2 bis 20 Kohlenstoff- atomen oder eine gegebenenfalls substituierte Cyclo- alkylen- oder Arylengruppe mit 6 bis 12 Kohlenstoffatomen oder eine Einfachbindung

bedeuten.

Beispiele für Y sind eine Einfachbindung, Methylen, 1,2-Ethylen, 1,3-Propylen, 1,4-Butylen, 1,6-Hexylen, 1,7-Heptylen, 1,8-Octylen, cis-1,2-Ethenylen, trans-1,2-Ethenylen, 1,2-, 1,3-oder 1,4-Phenylen, 1,2-Cyclohex-1-enylen, 1,2-, 1,3- oder 1,4-Cyclohexylen, 4-Carboxy-1,2-phenylen, 2-Carboxy-1,4-phenylen oder 1-Carboxy-2,4-phenylen.

Bevorzugte Gruppen Y sind 1,2-Ethylen, 1,4-Butylen und 1,2-, 1,3-oder 1,4-Phenylen.

Selbstverständlich liegen herstellungsbedingt in der Regel Gemische vor, in denen zusätzlich niedere und höhere Oligomere enthalten sein können.

In einer weiteren bevorzugten Ausführungsform werden Reaktionsgemische von zumindest teilweise verseiften Polyesterolen als Polyalkohole A zur Herstellung des Esters F eingesetzt.

Dazu werden die z.B. oben beschriebenen Polyesterole mit einer geeigneten Base zumindest teilweise verseift und anschließend, gegebenenfalls nach Abtrennung der im Reaktionsgemisch verbliebenen basischen Bestandteile, mit der Carbonsäure B verestert.

Geeignete Basen sind beispielsweise NaOH, KOH, $Ca(OH)_2$, Kalkmilch, $Na_2CO_3$ oder $K_2CO_3$, beispielsweise als Feststoff, Lösung oder Suspension, bevorzugt in Form einer 10 - 50 Gew%igen Lösung, besonders bevorzugt in Form einer 20-40 Gew%igen wäßrigen Lösung.

Die Verseifung, d.h. die Spaltung der im Polyesterol enthaltenen Estergruppen, erfolgt zu beispielsweise mindestens 10% bezogen auf die Estergruppen in der Ausgangsverbindung, bevorzugt zu mindestens 25%, besonders bevorzugt zu mindestens 50%, ganz besonders bevorzugt zu mindestens 75% und insbesondere zu mindestens 90 %.

Falls basische Bestandteile, wie z.B. das basische Salz der Carbonsäure, aus dem Reaktionsgemisch entfernt werden sollen, kann dies beispielsweise über Ionentauscher, z.B. saure oder stark saure Ionentauscher erfolgen.

Das Reaktionsgemisch wird anschließend angesäuert und mit der Carbonsäure B wie beschrieben verestert.

Polyester(meth)acrylate können in mehreren Stufen oder auch einstufig, wie z.B. in EP-A 279 303 beschrieben, aus (Meth)acrylsäure, Polycarbonsäure und Polyol hergestellt werden.

Ebenfalls als Polyalkohole einsetzbar sind alkoxylierte Polyole und Polyesterolev, die durch Umsetzung eines Polyols oder Polesterols mit mindestens einem Alkylenoxid erhältlich sind.

Erfindungsgemäß lassen sich auch solche Verbindungen der Formel VII enthaltende Reaktionsgemische darstellen.

$$R^8\text{- (O(CH (}R^{10}\text{) CH (}R^{10}\text{) O) y-C (=O) -R-}^9)_x \qquad \text{(VII)},$$

worin

- $R^8$ ein mehrwertiger, geradkettiger oder verzweigter $C_2$-$C_{10}$-Alkyl- rest,
- $R^9$ unabhängig voneinander ein geradkettiger oder verzweigter $C_2$-$C_{10}$-Alkenylrest,
- $R^{10}$ unabhängig voneinander Wasserstoff oder Methyl,
- x unabhängig voneinander eine positive ganze Zahl von 2 oder größer und
- y unabhängig voneinander für x=2 eine Zahl von 3 bis 8 und für x=3 oder größer eine Zahl von 2 bis 7 ist.

Der zugrundeliegende zu veresternde Alkohol weist dabei die Formel VIIa auf,

$$R^8\text{- (O (CH (}R^{10}\text{) CH(}R^{10}\text{) O) y-H)}_x \qquad \text{(VIIa)},$$

worin $R^8$, $R^{10}$, x und y wie oben definiert sind.

Die Verbindungen der Formel (VII) sind in der Regel 2 bis 10 Kohlenstoffatome aufweisende mehrwertige Alkohole VIIa, die mit zwischen 2 und 8 Alkylenoxideinheiten pro Hydroxygruppe alkoxyliert sind und deren endständige Hydroxygruppe jeder Alkylenoxidkette mit einer 2 bis 10 Kohlenstoffatome aufweisenden ungesättigten Carbonsäure oder deren Ester verestert ist. Bevorzugt handelt es sich bei dem Startalkohol um einen 3 - 6 Kohlenstoffatome aufweisenden mehrwertigen Alkohol, der bevorzugt 2 bis 4 Hydroxygruppen trägt. Besonders bevorzugt handelt es sich bei dem Startalkohol um Trimethylolpropan, Glycerin, Pentaerythrit, 1,3-Propandiol, Propylenglykol, 1,4-Butandiol oder Butylenglykol. Ganz besonders bevorzugt sind Trimethylolpropan, Glycerin und Pentaerythrit als Startalkohol.

Geeignete Alkylenoxide sind beispielsweise Ethylenoxid, Propylenoxid, *iso*-Butylenoxid, Vinyloxiran und/oder Styroloxid.

Die Alkylenoxidkette kann bevorzugt aus Ethylenoxid-, Propylenoxid- und/oder Butylenoxideinheiten zusammengesetzt sein. Eine solche Kette kann sich aus einer Spezies eines Alkylenoxides oder aus einem Gemisch von Alkylenoxiden zusammensetzen. Wird ein Gemisch verwendet, können die unterschiedlichen Alkylenoxideinheiten statistisch oder als Block oder Blöcke einzelner Spezies vorliegen. Bevorzugt ist als Alkylenoxid Ethylenoxid, Propylenoxid oder ein Gemisch daraus, besonders bevorzugt ist es Ethylenoxid oder Propylenoxid und ganz besonders bevorzugt Ethylenoxid. Somit ist bevorzugt ein Rest $R^{10}$ pro Alkylenoxideinheit Wasserstoff und der andere Methyl oder Wasserstoff, besonders bevorzugt sind beide Reste $R^{10}$ Wasserstoff.

Die bevorzugte Anzahl der Alkylenoxideinheiten in jeder Kette ist abhängig von der Anzahl der Ketten.

Das Veresterungsmittel ist eine 2 bis 10 Kohlenstoffatome aufweisende, geradkettige oder verzweigte ethylenisch ungesättigte Carbonsäure oder deren Ester, bevorzugt eine 2 bis 4 und besonders bevorzugt eine 2 bis 3 Kohlenstoffatome aufweisende ethylenisch ungesättigte Carbonsäure, ganz besonders bevorzugt Acrylsäure, Methacrylsäure oder deren Ester, insbesondere Acrylsäure.

Häufig liegen die Verbindungen der Formel VII als Gemisch von Verbindungen, die durch diese Formel beschrieben werden, und Nebenprodukten des Herstellungsprozesses vor.

Insbesondere bevorzugt sind unter diesen Verbindungen VII die je Hydroxygruppe bis zu sechsfach, besonders bevorzugt die bis zu vierfach und ganz besonders bevorzugt die vierfach ethoxylierten Verbindungen, im folgenden Verbindungen VIIb genannt. Diese weisen eine erhöhte Hydrolysestabilität auf.

Gleichermaßen bevorzugt sind Verbindungen VII, die je Hydroxygruppe

- für x=2 mehr als achtfach, besonders bevorzugt mehr als zehnfach, ganz besonders bevorzugt mehr als zwölffach und insbesondere mindestens 15fach, beziehungsweise
- für x=3 oder größer mehr als siebenfach, besonders bevorzugt mehr als neunfach, ganz besonders bevorzugt mehr als zwölffach und insbesondere mindestens 15fach ethoxyliert sind, im folgenden Verbindungen VIIc genannt, da diese in der Regel eine erhöhte Wasserlöslichkeit aufweisen.

Denkbar sind auch solche Verbindungen VII, bei denen für x = 2 y Werte von 0, 1 oder 2 und für x = 3 y Werte von 0 oder 1 annehmen kann.

Insbesondere sind Mischungen aus den Verbindungen VIIb und VIIc vorteilhaft, beispielsweise solche mit einem Gewichtsverhältnis VIIb:VIIc von 10:90 bis 90:10, bevorzugt 20:80 bis 80:20, besonders bevorzugt 30:70 bis 70:30 und ganz besonders bevorzugt von 40:60 bis 60:40.

Bevorzugte Beispiele für derartige alkoxylierte Polyole sind die Alkoxylierungsprodukte (IIa), (IIb) oder (IIc) von Polyolen der Formel (I),

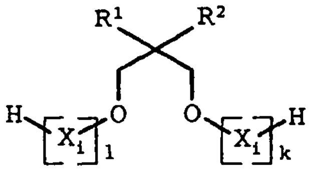

(IIa)

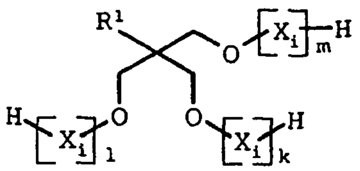

(IIb)

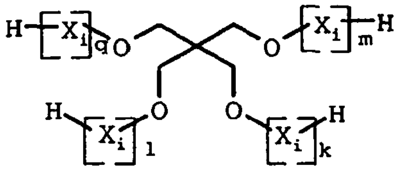

(IIc)

worin

| | |
|---|---|
| $R^1$, $R^2$ | die oben genannten Bedeutungen haben, |
| k, 1, m, q | unabhängig voneinander je für eine ganze Zahl von 1 bis 10, bevorzugt 1 bis 5, besonders bevorzugt 3 bis 5 und insbesondere 4 steht und |
| jedes $X_i$ | für i = 1 bis k, 1 bis 1, 1 bis m und 1 bis q unabhängig voneinander ausgewählt sein kann aus der Gruppe $-CH_2-CH_2-O-$, $-CH_2-CH(CH_3)-O-$, $-CH(CH_3)-CH_2-O-$, $-CH_2-C(CH_3)_2-O-$, $-C(CH_3)_2-CH_2-O-$, $-CH_2-CHVin-O-$, $-CHVin-CH_2-O-$, $-CH_2-CHPh-O-$ und $-CHPh-CH_2-O-$, bevor- zugt aus der Gruppe $-CH_2-CH_2-O-$, $-CH_2-CH(CH_3)-O-$ und $-CH(CH_3)-CH_2-O-$, und besonders bevorzugt $-CH_2-CH_2-O-$, |

worin Ph für Phenyl und Vin für Vinyl steht.

Bevorzugt handelt es sich dabei um ein- bis fünffach, besonders bevorzugt drei- bis fünffach und ganz besonders bevorzugt vierfach ethoxyliertes, propoxyliertes oder gemischt ethoxyliertes und propoxyliertes und insbesondere ausschließlich ethoxyliertes Neopentylglykol, Trimethylolpropan, Trimethylolethan oder Pentaerythrit.

Unter diesen besonders bevorzugt sind solche mehrwertigen Alkohole der Formel (IIb).

Gleichermaßen bevorzugt ist ein ein bis 20fach, bevorzugt ein bis zehnfach, besonders bevorzugt zwei bis zehnfach, ganz besonders bevorzugt zwei bis fünffach, insbesondere drei bis fünffach und speziell drei bis vierfach alkoxyliertes, bevorzugt ethoxyliertes, propoxyliertes oder gemischt-ethoxyliert-propoxyliertes und besonders bevorzugt ethoxyliertes Glycerin (hier ausnahmsweise berechnet in mol Alkoxygruppen pro mol Glycerin).

Die angegebenen Alkoxylierungsgrade beziehen sich dabei jeweils auf den mittleren Alkoxylierungsgrad.

Das zahlenmittlere Molgewicht $M_n$ der alkoxylierten Polyole beträgt bevorzugt nicht mehr als 1000 g/mol, besonders bevorzugt nicht mehr als 800 g/mol und ganz besonders bevorzugt nicht mehr als 550 g/mol.

Die Angaben zum zahlenmittleren und gewichtsmittleren Molekulargewicht $M_n$ und $M_w$ beziehen sich hier auf gelpermeationschromatographische Messungen, wobei Polystyrol als Standard und Tetrahydrofuran als Elutionsmittel verwendet wurde. Die Methode ist im Analytiker Taschenbuch Bd. 4, Seiten 433 bis 442 , Berlin 1984 beschrieben.

Beispiele für alkoxylierte Zuckeralkohole sind solche Verbindungen, die aus Zuckeralkoholen, beispielsweise aus den oben aufgeführten Zuckeralkoholen, durch Alkoxylierung, beispielsweise mit den oben aufgeführten Alkylenoxiden, bevorzugt mit Ethylenoxid und/oder Propylenoxid und ganz besonders bevorzugt mit Ethylenoxid erhältlich sind. Beispiele dafür sind

- die aufgeführten Tetrole, die im statistischen Mittel pro mol Zuckeralkohol 2 - 30fach, bevorzugt 2 - 20fach, besonders bevorzugt 3-10 fach und insbesondere 3, 4, 5, 6, 7 oder 8fach alkoxyliert sind,

- die aufgeführten Pentole, die im statistischen Mittel pro mol Zuckeralkohol 3 - 35fach, bevorzugt-3-28 fach, besonders bevorzugt 4-20fach und insbesondere 4, 5, 6, 7, 8, 9 oder 10fach alkoxyliert sind,

- höhere Zuckeralkohole, die im statistischen Mittel pro mol Zuckeralkohol 4-50fach, bevorzugt 6-40fach, besonders bevorzugt 7-30fach, ganz besonders bevorzugt 8-20fach und insbesondere 10-15 fach alkoxyliert sind.

Bevorzugte alkoxylierte Zuckeralkohole sind solche, bei denen mindestens eine Hydroxygruppe des Zuckeralkohols nicht alkoxyliert ist.

Bevorzugte Beispiele für alkoxylierte Polesterole sind solche der Formel (IVa-c),

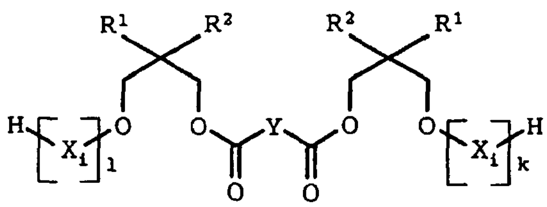

(IVa)

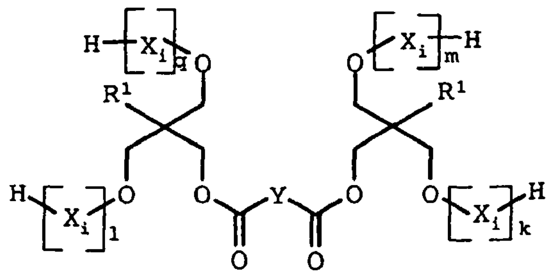

(IVb)

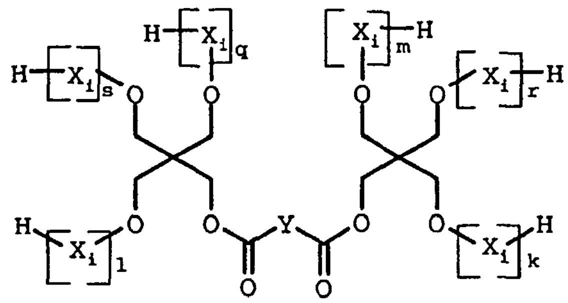

(IVc)

worin

| | |
|---|---|
| $R^1$,$R^2$,Y | die oben genannten Bedeutungen haben, |
| k, 1, m, q, r, s | unabhängig voneinander je für eine ganze Zahl von 1 bis 30, bevorzugt 1 bis 20, besonders bevorzugt 1 bis 10 und insbesondere 1 bis 5 steht und |
| jedes Xi | für i = 1 bis k, 1 bis 1, 1 bis m, 1 bis q, 1 bis r und 1 bis s unabhängig voneinander ausgewählt sein |

kann aus der Gruppe $-CH_2-CH_2-O-$, $-CH_2-CH(CH_3)-O-$, $-CH(CH_3)-CH_2-O-$, $-CH_2-C(CH_3)_2-O-$, $(C(CH_3)_2-CH_2-O-$, $-CH_2-CHVin-O-$, $-CHVin-CH_2-O-$, $-CH_2-CHPh-O-$ und $-CHPh-CH_2-O-$, bevorzugt aus der Gruppe $-CH_2-CH_2-O-$, $-CH_2-CH(CH_3)-O-$ und $-CH(CH_3)-CH_2-O-$, und besonders bevorzugt $-CH_2-CH_2-O-$,

worin Ph für Phenyl und Vin für Vinyl steht,
bedeuten.

Bevorzugt handelt es sich dabei um urialkoxyliertes oder ein- bis zehnfach, besonders bevorzugt zwei- bis fünffach ethoxyliertes, propoxyliertes oder gemischt ethoxyliertes und propoxyliertes, mit Adipinsäure, Phthalsäure, Terephthalsäure oder Isophthalsäure verstertes Neopentylglykol, Trimethylolpropan, Trimethylolethan oder Pentaerythrit.

Die Umsetzung der Alkohole mit einem Alkylenoxid ist dem Fachmann an sich bekannt. Mögliche Durchführungsformen finden sich in Houben-Weyl, Methoden der Organischen Chemie, 4. Auflage, 1979, Thieme Verlag Stuttgart, Hrsg. Heinz Kropf, Band 6/1a, Teil 1, Seiten 373 bis 385.

Werden gemischt alkoxylierte Alkohole verwendet, so können die darin enthaltenen unterschiedlichen Alkoxygruppen zueinander im molaren Verhältnis von beispielsweise 0,05 - 20 : 1, bevorzugt 0,1 - 10 : 1 und besonders bevorzugt 0,2 - 5 : 1 stehen.

An die Viskosität der erfindungsgemäß einsetzbaren Polyalkohole werden keine besonderen Ansprüche gestellt, außer daß sie bei einer Temperatur bis zu ca. 80 °C problemlos pumpbar sein sollten, bevorzugt sollten sie eine Viskosität unter 1000 mPas aufweisen, bevorzugt unter 800 mPas und ganz besonders bevorzugt unter 500 mPas.

Werden als Polyalkohole drei- oder höherwertige Polyalkohole in die Veresterung eingesetzt, so kann es für deren erfindungsgemäße Verwendung als Radikalvernetzer sinnvoll sein, die Polyalkohole lediglich teilzuverestern. Dies bedeutet, daß bei einem n-wertigen Polyalkohol lediglich mindestens 2 der n Hydroxygruppen mit der Carbonsäure B verestert werden.

Für n=3 beträgt der Veresterungsgrad mindestens 2, für n=4 mindestens 2, bevorzugt mindestens 2,5 und besonders bevorzugt mindestens 3, für n=5 oder größer mindestens 2, bevorzugt mindestens 3 und besonders bevorzugt mindestens 4.

In einem solchen Fall berechnet sich der einzusetzende stöchiometrische Überschuß an Carbonsäure B auf den angestrebten Veresterungsgrad, beträgt also beispielsweise das 2/n-fache der oben angegebenen molaren Überschüsse. Selbstverständlich kann die Veresterung auch, z.B. durch Kühlung oder Verdünnung, abgebrochen werden, wenn der gewünschte Veresterungsgrad erreicht ist.

Erfindungsgemäß einsetzbare ethylenisch ungesättigte Carbonsäuren B sind solche Verbindungen, die mindestens eine Carboxylgruppe (-COOH), bevorzugt eine, und mindestens eine, bevorzugt eine, ethylenisch ungesättigte Gruppe aufweisen.

Die erfindungsgemäß einsetzbaren Carbonsäuren können aliphatisch, cycloaliphatisch oder aromatisch sein, bevorzugt aliphatisch oder cycloaliphatisch und ganz besonders bevorzugt aliphatisch, geradkettig oder verzweigt und gegebenenfalls substituiert mit funktionellen Gruppen.

In der Regel weisen die Carbonsäuren drei bis zehn Kohlenstoffatome auf, bevorzugt drei bis fünf und besonders bevorzugt drei bis vier.

Beispiele für ethylenisch ungesättigte Carbonsäuren B sind Acrylsäure, Methacrylsäure, Ethacrylsäure, Maleinsäure einschließlich deren Anhydrid, Fumarsäure, Itaconsäure, Citraconsäure, Mesaconsäure, Vinylessigsäure, Allylessigsäure oder Crotonsäure.

Bevorzugte Carbonsäuren B sind $\alpha,\beta$-ungesättigte Carbonsäuren.

Besonders bevorzugt sind Methacrylsäure und Acrylsäure, in dieser Schrift (Meth)acrylsäure genannt, ganz besonders bevorzugt ist Acrylsäure.

Erfindungsgemäß einsetzbare Veresterungskatalysatoren C sind Schwefelsäure, Aryl- oder Alkylsulfonsäuren oder Gemische davon. Beispiele für Arylsulfonsäuren sind Benzolsulfonsäure, para-Toluolsulfonsäure oder Dodecylbenzolsulfonsäure, Beispiele für Alkylsulfonsäuren sind Methansulfonsäure, Ethansulfonsäure oder Trifluormethansulfonsäure. Auch stark saure Ionentauscher oder Zeolithe sind als Veresterungskatalysatoren einsetzbar. Bevorzugt sind Schwefelsäure und Ionentauscher.

Erfindungsgemäß einsetzbare Polymerisationsinhibitoren D sind beispielsweise Phenole wie Alkylphenole, beispielsweise o-, m-oder p-Kresol (Methylphenol), 2-tert.-Butyl-4-methylphenol, 6-tert.-Butyl-2,4-dimethyl-phenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol), 4,4'-0xydiphenyl, 3,4-Methylendioxydiphenol (Sesamol), 3,4-Dimethylphenol, Hydrochinon, Brenzcatechin (1,2-Dihydroxybenzol), 2-(1'-Methylcyclohex-1'-yl)-4,6-dimethylphenol, 2- oder 4-(1'-Phenyl-eth-1'-yl)-phenol , 2-tert-Butyl-6-methylphenol , 2 ,4 ,6-Tris-tert-Butylphenol, 2,6-Di-tert.-butylphenol, 2,4-Di-tert.-butylphenol, 4-tert.-Butylphenol , Nonylphenol [11066-49-2] , Octylphenol [140-66-9] , 2 ,6-Dimethylphenol, Bisphenol A , Bisphenol F , Bisphenol B, Bisphenol C , Bisphenol S , 3,3c' ,5,5'-Tetrabromobisphenol A, 2,6-Di-tert-Butyl-p-kresol, Koresin® der BASF AG, 3,5-Di-tert-Butyl-4-hydroxybenzoesäuremethylester, 4-tert-Butylbrenzcatechin, 2-Hydroxybenzylalkohol, 2-Methoxy-4-methylphenol, 2,3,6-Trimethylphenol, 2,4,5-Trimethylphenol, 2,4,6-Trimethylphenol, 2-Isopropylphenol, 4-Isopropylphenol, 6-Isopropyl-m-Kresol, n-Octadecyl-β-(3,5-di-tert-butyl-4-hydroxyphenyl)propionat, 1,1,3-Tris-(2-methyl-4-hydroxy-5-tert-butylphenyl)butan, 1,3,5-Trimethyl-2,4,6-tris-(3,5-di-tert-butyl-4-hydroxybenzyl) benzol, 1,3,5,-Tris-(3,5-di-tert-butyl-4-hydroxybenzyl)isocyanurat, 1,3,5,-Tris-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat oder Pentaerythrit-tetrakis-[β-(3,5,-di-tert-butyl-4-hydroxyphenyl)-propionat], 2,6-Di-tert.-butyl-4-dimethylaminomethyl-phenol, 6-*sek.*-Butyl- 2,4-dinitrophenol, Irganox® 565, 1141, 1192, 1222 und 1425 der Firma Ciba Spezialitätenchemie, 3-(3',5'-Di-*tert.*-butyl-4'-hydroxyphenyl) propionsäureoctadecylester, 3-(3',5'-Di-*tert.*-butyl-4'-hydroxyphenyl)propionsäurehexadecylester, 3-(3',5'-Di-tert.-butyl-4'-hydroxyphenyl)propionsäureoctylester, 3-Thia-1,5-pentandiol-bis-[(3',5'-di-tert.-butyl-4'-hydroxyphenyl) propionat], 4,8-Dioxa-1,11-undecandiolbis-[(3',5'-di-*tert.*-butyl-4'-hydroxyphenyl)propionat], 4,8-Dioxa-1,11-undecandiol-bis-[(3'-*tert.*-butyl-4'-hydroxy-5'-methylphenyl)propionat], 1,9-Nonandiol-bis-[(3',5'-di-*tert.*-butyl-4'-hydroxyphenyl)propionat], 1,7-Heptandiamin-bis[3-(3',5'-di-*tert.*-butyl-4'-hydroxyphenyl)propionsäureamid], 1,1-Methandiamin-bis[3-(3',5'-di-*tert.*-butyl-4'-hydroxyphenyl)propionsäureamid], 3-(3',5'-di-*tert.*-Butyl-4'-hydroxyphenyl) propionsäurehydrazid, 3-(3',5'-di-Methyl-4'-hydroxyphenyl)propionsäurehydrazid, Bis(3-*tert.*-Butyl-5-ethyl-2-hydroxy-phen-1-yl)methan, Bis(3,5-di-*tert.*-Butyl-4-hydroxyphen-1-yl)methan, Bis[3-(1'-methylcyclohex-1'-yl)-5-methyl-2-hydroxy-phen-1-yl]methan, Bis(3-*tert.*-Butyl-2-hydroxy-5-methyl-phen-1-yl)methan, 1,1-Bis(5-*tert.*-Butyl-4-hydroxy-2-methyl-phen-1-yl)ethan, Bis(5-*tert.*-Butyl-4-hydroxy-2-methyl-phen-1-yl)sulfid, Bis(3-tert.-Butyl-2-hydroxy-S-methyl-phen-1-yl)sulfid, 1,1-Bis(3,4-Dimethyl-2-hydroxy-phen-1-yl)-2-methylpropan, 1,1-Bis(5-*tert.*-Butyl-3-methyl-2-hydroxy-phen-1-yl)-butan, 1,3,5-Tris[1'-(",5"-di-tert.-Butyl-4"-hydroxyphen-1"-yl)-meth-1'-yl]-2,4,6-trimethylbenzol, 1,1,4-Tris(5'-*tert.*-Butyl-4'-hydroxy-2'-methyl-phen-1'-yl)butan, Aminophenole, wie z.B. para-Aminophenol, Nitrosophenole, wie z.B. para-Nitrosophenol, p-Nitroso-o-Kresol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, 3,5-Di-tert-butyl-4-hydroxyanisol, 3-Hydroxy-4-methoxybenzylalkohol, 2,5-Dimethoxy-4-hydroxybenzylalkohol (Syringaalkohol), 4-Hydroxy-3-methoxybenzaldehyd (Vanillin), 4-Hydroxy-3-ethoxybenzaldehyd (Ethylvanillin), 3-Hydroxy-4-methoxybenzaldehyd (Isovanillin), 1-(4-Hydroxy-3-methoxy-phenyl)ethanon (Acetovanillon), Eugenol, Dihydroeugenol, Isoeugenol, Tocopherole, wie z.B. α-, β-, γ-, δ- und ε-Tocopherol, Tocol, α-Tocopherolhydrochinon, sowie 2,3-Dihydro-2,2-dimethyl-7-hydroxybenzofuran (2,2-Dimethyl-7-hydroxycumaran), Chinone und Hydrochinone wie Hydrochinon oder Hydrochinonmonomethylether, 2,5-Di-tert.-Butylhydrochinon, 2-Methyl-p-hydrochinon, 2,3-Dimethylhydrochinon, Trimethylhydrochinon, 4-Methylbrenzcatechin, tert-Butylhydrochinon, 3-Methylbrenzcatechin, Benzochinon, 2-Methyl-p-hydrochinon, 2,3-Dimethylhydrochinon, Trimethylhydrochinon, 3-Methylbrenzcatechin; 4-Methylbrenzcatechin, tert-Butylhydrochinon, 4-Ethoxyphenol, 4-Butoxyphenol, Hydrochinonmonobenzylether, p-Phenoxyphenol, 2-Methylhydrochinon, 2,5-Di-tert.-Butylhydrochinon, Tetramethyl-p-benzochinon, Diethyl-1,4-cyclohexandion-2,5-dicarboxylat, Phenyl-p-benzochinon, 2,5-Dimethyl-3-benzyl-p-benzochinon, 2-Isopropyl-5-methyl-p-benzochinon (Thymochinon), 2,6-Diisopropyl-p-benzochinon, 2,5-Dimethyl-3-hydroxy-p-benzochinon, 2,5-Dihydroxyp-benzochinon, Embelin, Tetrahydroxy-p-benzochinon, 2,5-Dimethoxy-1,4-benzochinon, 2-Amino-5-methyl-p-benzochinon, 2,5-Bisphenylamino-1,4-benzochinon, 5,8-Dihydroxy-1,4-naphthochinon, 2-Anilino-1,4,naphthochinon, Anthrachinon, N,N-Dimethylindoanilin, N,N-Diphenyl-p-benzochinondiimin, 1,4-Benzochinondioxim, Coerulignon, 3,3'-Di-tert-butyl-5,5'-dimethyldiphenochinon, p-Rosolsäure (Aurin), 2,6-Di-tert-butyl-4-benzyliden-benzochinon, 2,5-Di-tert.-Amylhydrochinon, N-Oxyle wie 4-Hydroxy-2,2,6,6-tetramethylpiperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl, 4,4',4"-Tris(2,2,6,6-tetramethyl-piperidin-N-oxyl)-phosphit, 3-Oxo-2,2,5,5-tetramethyl-pyrrolidin-N-oxyl, 1-Oxyl-2,2,6,6-tetramethyl-4-methoxypiperidin, 1-Oxyl-2,2,6,6-tetramethyl-4-trimethylsilyloxypiperidin, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis(1-Oxyl-2,2,6,6-tetramethylpiperidin-4-yl)-succinat, Bis(1-Oxyl-2,2,6,6-tetramethylpiperidin-4-yl)-adipat, 1,10-Dekandisäure-bis(1-Oxyl-2,2,6,6-tetramethylpiperidin-4-yl)ester, Bis(1-Oxyl-2,2,6,6-tetramethylpiperidin-4-yl)-n-butylmalonat, Bis(1-Oxyl-2,2,6,6-tetramethylpiperidin-4-yl)-phthalat, Bis(1-Oxyl-2,2,6,6-tetramethylpiperidin-4-yl)-isophthalat, Bis(1-Oxyl-2,2,6,6-tetramethylpiperidin-4-yl)-terephthalat, Bis(1-Oxyl-2,2,6,6-tetramethylpiperidin-4-yl)-hexahydroterephthalat, N,N'-Bis(1-Oxyl-2,2,6,6-tetramethylpiperidin-4-yl)-adipinamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-Tris-[N-butyl-N-(1-Oxyl-2,2,6,6-tetramethylpiperidin-4-yl]triazin, N,N'-Bis(1-Oxyl-2,2,6,6-tetramethylpiperidin-4-yl)-N,N'-bis-formyl-1,6-diaminohexan, 4,4'-Ethylenbis(1-Oxyl-2,2,6,6-tetramethylpiperazin-3-on) aromatische Amine wie Phenylendiamine, N,N-Diphenylamin, N-Nitroso-diphenylamin, Nitrosodiethylanilin, N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, beispielsweise N,N'-Di-iso-butyl-p-phenylendiamin, Nv,N'-Di-isopropyl-p-phenylendiamin, Irganox 5057 der Firma Ciba Spezialitätenchemie, N,N'-Di-iso-butyl-p-phenylendiamin, N,N'-Di-iso-propyl-p-phenylendiamin, p-Phenylendiamin, N-Phenyl-p-Phenylendiamin, N,N'-Diphenyl-p-phenylendiamin, N-Isopropyl-N-phenyl-p-phenylendiamin, N,N'Di-sec-butyl-p-phenylendiamin (Kerobit® BPD der BASF AG), N-Phenyl-N'-isopropyl-p-phenylendiamin (Vulkanox® 4010 der Bayer AG), N-(1,3-Dimethylbutyl)-N'-phenyl-p-phenylendiamin, N-Phenyl-2-naphthylamin, Imoinodibenzyl, N,N'-Diphenylbenzidin, N-Phenyltetraanilin, Acridon, 3-Hydroxydiphenylamin, 4-Hydroxydiphenylamin, Hydroxylamine wie N,N-Diethylhydroxylamin, Harnstoffderivate wie Harnstoff oder Thioharnstoff, phosphorhaltige Verbindungen, wie Triphenylphosphin, Triphenylphosphit, Hypophosphorige Säure oder Triethylphosphit, schwefelhaltige Verbindungen, wie Diphenylsulfid, Phenothiazin oder Metallsalze, wiebeispielsweise Kupfer-, Mangan-, Cer-, Nickel-, Chrom--chlorid, -dithiocarbamat, -sulfat, -salicylat oder -acetat oder Gemische davon. Bevorzugt sind die genannten Phenole und Chinone, besonders bevorzugt sind Hydrochinon, Hydrochinonmonomethylether, 2-tert.-Butyl-4-methylphenol, 6-tert.-Butyl-2,4-dimethyl-phenol, 2,6-Di-tert.-Butyl-4-methylphenol, 2,4-di-tert.-Butylphenol, Triphenylphosphit, Hypophosphorige Säure, $CuC1_2$ und Guajacol, ganz besonders bevorzugt sind Hydrochinon und Hydrochinonmonomethylether.

Besonders bevorzugt sind Hydrochinonmonomethylether, Hydrochinon, und Alkylphenole, gegebenenfalls in Kombination mit Tripehnylphosphit und/oder Hypophosphoriger Säure.

Zur weiteren Unterstützung der Stabilisierung kann ein sauerstoffhaltiges Gas, bevorzugt Luft oder ein Gemisch aus Luft und Stickstoff (Magerluft) anwesend sein.

Unter den aufgeführten Stabilisatoren sind solche bevorzugt, die aerob sind, d.h. solche, die zur Entfaltung ihrer vollen Inhibitorwirkung die Anwesenheit von Sauerstoff erfordern.

Erfindungsgemäß einsetzbare Lösungsmittel E sind besonders solche, die sich zur azeotropen Entfernung des Reaktionswassers, falls gewünscht, eignen, vor allem aliphatische, cycloaliphatische und aromatische Kohlenwasserstoffe oder Gemische davon.

Vorzugsweise kommen n-Pentan, n-Hexan, n-Heptan, Cyclohexan, Methylcyclohexan, Benzol, Toluol oder Xylol zur Anwendung. Besonders bevorzugt sind Cyclohexan, Methylcyclohexan und Toluol.

Für die Veresterung können die dem Fachmann bekannten Herstell- und/oder Aufarbeitungsverfahren von mehrwertigen Alkoholen angewendet werden, beispielsweise die eingangs erwähnten oder die in DE-A 199 41 136, DE-A 38 43 843, DE-A 38 43 854, DE-A 199 37 911, DE-A 199 29 258, EP-A 331 845, EP 554 651 oder US 4 187 383 beschriebenen.

Im allgemeinen kann die Veresterung wie folgt durchgeführt werden:

Die Veresterungsapparatur besteht aus einem gerührten Reaktor, bevorzugt aus einem Reaktor mit Umlaufverdampfer und einer aufgesetzten Destillationseinheit mit Kondensator und Phasentrenngefäß.

Bei dem Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf, d.h. unter Verwendung einer Pumpe, besonders bevorzugt Naturumlauf, bei dem der Kreislaufstrom ohne mechanische Hilfsmittel bewerkstelligt wird, eingesetzt.

Selbstverständlich kann die Reaktion auch in mehreren Reaktionszonen, beispielsweise einer Reaktorkaskade aus zwei bis vier, bevorzugt zwei bis drei Reaktoren durchgeführt werden.

Geeignete Umlaufverdampfer sind dem Fachmann bekannt und beispielsweise beschrieben in R. Billet, Verdampfertechnik, HTB-Verlag, Bibliographisches Institut Mannheim, 1965, 53. Beispiele für Umlaufverdampfer sind Rohrbündelwärmetauscher, Plattenwärmetauscher, etc.

Selbstverständlich können im Umlauf auch mehrere Wärmetauscher vorhanden sein.

Die Destillationseinheit ist von an sich bekannter Bauart. Dabei kann es sich um eine einfache Destillation handeln, die gegebenenfalls mit einem Spritzschutz ausgestattet ist, oder um eine Rektifikationskolonne. Als Kolonneneinbauten kommen prinzipiell alle gängigen Einbauten in Betracht, beispielsweise Böden, Pakkungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

In der Regel sind 5 bis 20 theoretische Böden ausreichend.

Der Kondensator und das Trenngefäß sind von herkömmlicher Bauart.

Carbonsäure B und Polyalkohol A werden in der Veresterung a) in der Regel im molaren Überschuß wie oben angegeben bezogen auf die Hydroxygruppen des Alkohols eingesetzt. Der eingesetzte Überschuß kann bis zu ca. 1000: 1 betragen, falls gewünscht.

Als Veresterungskatalysatoren C kommen die oben angeführten in Frage.

Sie werden in der Regel in einer Menge von 0,1 - 5 Gew%, bezogen auf das Veresterungsgemisch, eingesetzt, bevorzugt 0,5 - 5, besonders bevorzugt 1 - 4 und ganz besonders bevorzugt 2 - 4 Gew%.

Falls erforderlich kann der Veresterungskatalysator aus dem Reaktionsgemisch mit Hilfe eines Ionenaustauschers entfernt werden. Der Ionenaustauscher kann dabei direkt in das Reaktionsgemisch gegeben und anschließend abfiltriert oder das Reaktionsgemisch kann über eine Ionenaustauscherschüttung geleitet werden.

Bevorzugt wird der Veresterungskatalysator im Reaktionsgemisch belassen. Handelt es sich jedoch bei dem Katalysator um einen Ionentauscher, so wird dieser bevorzugt entfernt, beispielsweise durch Filtration.

Zur weiteren Unterstützung der Stabilisierung kann ein sauerstoffhaltiges Gas, bevorzugt Luft oder ein Gemisch aus Luft und Stickstoff (Magerluft) anwesend sein.

Dieses sauerstoffhaltige Gas wird vorzugsweise in den Sumpfbereich einer Kolonne und/oder in einen Umlaufverdampfer eindosiert und/oder durch das Reaktionsgemisch und/oder über dieses geleitet.

Das Polymerisationsinhibitor(gemisch) D (wie oben angeführt) wird in einer Gesamtmenge von 0,01 - 1 Gew%, bezogen auf das Veresterungsgemisch, eingesetzt, bevorzugt 0,02 - 0,8, besonders bevorzugt 0,05 - 0,5 Gew%.

Das Polymerisationsinhibitor(gemisch) D kann beispielsweise als wäßrige Lösung oder als Lösung in einem Edukt oder Produkt eingesetzt werden.

b) Das bei der Reaktion entstehende Reaktionswasser kann während oder nach der Veresterung a) abdestilliert werden, wobei dieser Vorgang durch ein mit Wasser ein Azeotrop bildendes Lösungsmittel unterstützt werden kann.

Als Lösungsmittel E zur azeotropen Entfernung des Reaktionswassers, falls gewünscht, eignen sich die oben angeführten Verbindungen.

Bevorzugt ist die Durchführung der Veresterung in Gegenwart eines Lösungsmittels.

Die eingesetzte Menge an Lösungsmittel beträgt 10 - 200 Gew%, vorzugsweise 20 - 100 Gew%, besonders bevorzugt 30 bis 100 Gew% bezogen auf die Summe von Polyalkohol und Carbonsäure B.

Denkbar ist jedoch auch eine Durchführung ohne Schleppmittel, wie sie z.B. in der DE-A1 38 43 854, Sp. 2, Z. 18 bis Sp. 4, Z. 45 beschrieben ist, jedoch im Unterschied dazu mit den oben genannten Stabilisatoren.

Falls das im Reaktionsgemisch enthaltene Wasser nicht über ein azeotropbildendes Lösungsmittel entfernt wird, so ist es möglich, dieses über Strippen mit einem inerten Gas, bevorzugt einem sauerstoffhaltigen Gas, besonders bevorzugt mit Luft oder Magerluft zu entfernen, beispielsweise wie in der DE-A 38 43 843 beschrieben.

Die Reaktionstemperatur der Veresterung a) liegt allgemein bei 40 - 160 °C, bevorzugt 60 - 140°C und besonders bevorzugt 80 - 120°C. Die Temperatur kann im Reaktionsverlauf gleichbleiben oder ansteigen, bevorzugt wird sie im Reaktionsverlauf angehoben. In diesem Fall ist die Endtemperatur der Veresterung um 5 - 30 °C höher als die Anfangstemperatur. Die Temperatur der Veresterung kann durch Variation der Lösungsmittelkonzentration im Reaktionsgemisch bestimmt und geregelt werden, wie in DE-A 199 41 136 und der deutschen Anmeldung mit dem Aktenzeichen 100 63 175.4 beschrieben.

Falls ein Lösungsmittel eingesetzt wird, so kann dieses über die dem Reaktor aufgesetzte Destillationseinheit vom Reaktionsgemisch abdestilliert werden.

Das Destillat kann wahlweise entfernt werden, oder, nach Kondensation, in einen Phasentrennapparat geführt werden. Die so erhaltene wäßrige Phase wird in der Regel ausgeschleust, die organische Phase kann als Rücklauf in die Destillationseinheit geführt und/oder direkt in die Reaktionszone geleitet werden und/ oder in einen Umlaufverdampfer geführt werden, wie in der deutschen Patentanmeldung mit dem Aktenzeichen 100 63 175.4 beschrieben.

Bei Verwendung als Rücklauf kann die organische Phase, wie in der DE-A 199 41 136 beschrieben, zur Steuerung der Temperatur in der Veresterung verwendet werden.

Die Veresterung a) kann drucklos aber auch bei Überdruck oder Unterdruck durchgeführt werden, vorzugsweise wird bei normalem Druck gearbeitet.

Die Reaktionszeit beträgt in der Regel 2 - 20 Stunden, vorzugsweise 4 - 15 und besonders bevorzugt 7 bis 12 Stunden.

Die Reihenfolge der Zugabe der einzelnen Reaktionskomponenten ist erfindungsgemäß nicht wesentlich. Es können alle Komponenten gemischt vorgelegt und anschließend aufgeheizt werden oder es können eine oder mehrere Komponenten nicht oder nur teilweise vorgelegt und erst nach dem Aufheizen zugegeben werden.

Die einsetzbare Carbonsäure B ist in ihrer Zusammensetzung nicht beschränkt und kann im Fall von Roh-(Meth) acrylsäure beispielsweise folgende Komponenten aufweisen:

| | |
|---|---|
| (Meth)acrylsäure | 90 - 99,9 Gew% |
| Essigsäure | 0,05 - 3 Gew% |
| Propionsäure | 0,01 - 1 Gew% |
| Diacrylsäure | 0,01 - 5 Gew% |
| Wasser | 0,05 - 5 Gew% |
| Carbonylhaltige | 0,01 - 0,3 Gew% |
| Inhibitoren | 0,01 - 0,1 Gew% |
| Maleinsäure(-anhydrid) | 0,001 - 0,5 Gew% |

Die eingesetzte Roh-(Meth)acrylsäure ist in der Regel stabilisiert mit 200 - 600 ppm Phenothiazin oder anderen

Stabilisatoren in Mengen, die eine vergleichbare Stabilisierung ermöglichen. Unter dem Ausfruck Carbonylhaltige werden hier beispielsweise Aceton und niedere Aldehyde, wie z.B. Formaldehyd, Acetaldehyd, Crotonaldehyd, Acrolein, 2- und 3- Furfural und Benazaldehyd, verstanden.

Unter Roh-(Meth)acrylsäure wird hier das (meth)acrylsäurehaltige Gemisch verstanden, das nach Absorption der Reaktionsgase der Propan/Propen/Acrolein- beziehungsweise Isobutan/Isobuten/Methacrolein-Oxidation in einem Absorptionsmittel und anschließender Abtrennung des Absorptionsmittels anfällt beziehungsweise das durch fraktionierende Kondensation der Reaktionsgase gewonnen wird.

Selbstverständlich kann auch Rein-(Meth)acrylsäure eingesetzt werden mit beispielsweise folgender Reinheit:

| | |
|---|---|
| (Meth)acrylsäure | 99,7 - 99,99 Gew% |
| Essigsäure | 50 - 1000 Gew.ppm |
| Propionsäure | 10 - 500 Gew.ppm |
| Diacrylsäure | 10 - 500 Gew.ppm |
| Wasser | 50 - 1000 Gew.ppm |
| Carbonylhaltige | 1 - 500 Gew.ppm |
| Inhibitoren | 1 - 300 Gew.ppm |
| Maleinsäure(-anhydrid) | 1 - 200 Gew.ppm |

Die eingesetzte Rein-(Meth)acrylsäure ist in der Regel stabilisiert mit 100 - 300 ppm Hydrochinonmonomethylether oder anderen Lagerstabilisatoren in Mengen, die eine vergleichbare Stabilisierung ermöglichen.

Unter reiner bzw. vorgereinigter (Meth)acrylsäure wird allgemein (Meth)acrylsäure verstanden, deren Reinheit mind. 99,5 Gew% beträgt und die im wesentlichen frei von den aldehydischen, anderen carbonylhaltigen und hochsiedenden Komponenten ist.

Die während der Veresterung abdestillierte wäßrige Phase des über die aufgesetzte Kolonne, wenn vorhanden, abgetrennten Kondensats, die in der Regel 0,1 - 10 Gew% Carbonsäure B, beispielsweise (Meth)acrylsäure anthalten kann, wird abgetrennt und ausgeschleust. Vorteilhaft kann die darin enthaltene Carbonsäure, beispielsweise (Meth) acrylsäure mit einem Extraktionsmittel, bevorzugt dem gegebenenfalls in der Veresterung verwendeten Lösungsmittel, beispielsweise mit Cyclohexan, bei einer Temperatur zwischen 10 und 40 °C und einem Verhältnis von wäßriger Phase zu Extraktionsmittel von 1 : 5 - 30, bevorzugt 1 : 10 - 20, extrahiert und in die Veresterung zurückgeführt werden.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) in den Umlauf, durch oder über das Reaktionsgemisch geleitet werden, beispielsweise in Mengen von 0,1 - 1, bevorzugt 0,2 - 0,8 und besonders bevorzugt 0,3 - 0,7 m3/m3h, bezogen auf das Volumen des Reaktionsgemisches.

Der Verlauf der Veresterung a) kann durch Verfolgung der ausgetragenen Wassermenge und/oder die Abnahme der Carbonsäurekonzentration im Reaktor verfolgt werden.

Die Reaktion kann beispielsweise beendet werden, sobald 90 % der theoretisch zu erwartenden Wassermenge durch das Lösungsmittel ausgetragen worden sind, bevorzugt bei mindestens 95% und besonders bevorzugt bei mindestens 98%.

Das Ende der Reaktion kann beispielsweise dadurch festgestellt werden, daß im wesentlichen kein weiteres Reaktionswasser mehr über das Schleppmittel enfernt wird. Wird Carbonsäure B zusammen mit dem Reaktionswasser ausgetragen, so ist deren Anteil beispielsweise durch Rücktitration eines Aliquots der wäßrigen Phase bestimmbar.

Auf ein Entfernen des Reaktionswasers kann beispielsweise verzichtet werden, wenn die Carbonsäure B in einem hohen stöchiometrischen Überschuß eingesetzt wird, beispielsweise von mindestens 1,5:1, bevorzugt mindestens 2,5:1 und ganz besonders bevorzugt mindestens 5:1. In diesem Fall verbleibt ein wesentlicher Teil der entstandenen Wassermenge im Reaktionsgemisch. Während oder nach der Reaktion wird lediglich der Anteil an Wasser aus dem Reaktionsgemisch entfernt, der durch die Flüchtigkeit bei der angelegten Temperatur bestimmt ist und es werden darüberhinaus keine Maßnahmen zur Abtrennung des entstandenen Reaktionswassers durchgeführt. So können beispielsweise mindestens 10 Gew% des entstandenen Reaktionswassers im Reaktionsgemisch verbleiben, bevorzugt mindestens 20 Gew%, besonders bevorzugt mindestens 30 Gew%, ganz besonders bevorzugt mindestens 40 und insbesondere mindestens 50 Gew%.

c) Nach Beendigung der Veresterung kann das Reaktorgemisch auf übliche Weise auf eine Temperatur von 10 bis 30 °C abgekühlt werden und gegebenenfalls durch Zugabe von Lösungsmittel, das das gleiche wie das gegebenenfalls zur azeotropen Entfernung von Wasser verwendete Lösungsmittel oder ein anderes sein kann, eine beliebige Zielesterkonzentration eingestellt werden.

In einer weiteren Ausführungsform kann die Reaktion mit einem geeigneten Verdünnungsmittel G gestoppt werden und auf eine Konzentration von beispielsweise 10 - 90 Gew%, bevorzugt 20 - 80 %, besonders bevorzugt 20 bis 60%, ganz besonders bevorzugt 30 bis 50% und insbesondere ca. 40% verdünnt, beispielsweise um die Viskosität zu verringern.

Wichtig ist dabei, daß sich nach Verdünnung eine im wesentlichen homogene Lösung bildet.

Dies erfolgt bevorzugt erst relativ kurz vor dem Einsatz in der Herstellung des Hydrogels, beispielsweise nicht mehr als 24 Stunden vorher, bevorzugt nicht mehr als 20, besonders bevorzugt nicht mehr als 12, ganz besonders bevorzugt nicht mehr als 6 und insbesondere nicht mehr als 3 Stunden vorher.

Das Verdünnungsmittel G ist ausgewählt aus der Gruppe bestehend aus Wasser, ein Gemisch von Wasser mit einem oder mehreren in Wasser unbegrenzt löslichen organischen Lösemittel oder ein Gemisch von Wasser mit einem oder mehreren einfachen oder mehrfachfunktionellen Alkoholen, z.B. Methanol und Glycerin. Die Alkohole tragen bevorzugt 1, 2 oder 3 Hydroxygruppen und weisen bevorzugt zwischen 1 bis 10, insbesondere bis 4 C-Atome auf. Bevorzugt sind primäre und sekundäre Alkohole.

Bevorzugte Alkohole sind Methanol, Ethanol, Isopropanol, Ethylenglykol, 1,2-Propandiol oder 1,3-Propandiol.

d) Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1 - 10 Gew% bei Temperaturen von beispielsweise 10 bis 100 °C, bevorzugt 20 bis 80 °C und besonders bevorzugt 30 bis 60 °C unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

Die Entfärbung des Reaktionsgemisches kann an beliebiger Stelle des Aufarbeitungsverfahrens erfolgen, beispielsweise auf der Stufe des rohen Reaktionsgemisches oder nach gegebenenfalls erfolgter Vorwäsche, Neutralisation, Wäsche oder Lösungsmittelentfernung.

Das Reaktionsgemisch kann weiterhin einer Vorwäsche e) und/oder einer Neutralisation f) und/oder einer Nachwäsche g) unterworfen werden, bevorzugt lediglich einer Neutralisation f). Gegebenenfalls können Neutralisation f) und Vorwäsche e) in der Reihenfolge auch vertauscht werden.

Aus der wäßrigen Phase der Wäschen e) und g) und/oder Neutralisation f) kann enthaltene Carbonsäure B, beispielsweise (Meth)acrylsäure und/oder Katalysator C durch Ansäuern und Extraktion mit einem Lösungsmittel zumindest teilweise wiedergewonnen und von Neuem eingesetzt werden.

Zur Vor- oder Nachwäsche e) oder g) wird das Reaktionsgemisch in einem Waschapparat mit einer Waschflüssigkeit, beispielsweise Wasser oder einer 5 - 30 Gew%-igen, bevorzugt 5 - 20, besonders bevorzugt 5 - 15 Gew%-igen Kochsalz-, Kaliumchlorid-, Ammoniumchlorid-, Natriumsulfat- oder Ammoniumsulfatlösung, bevorzugt Wasser oder Kochsalzlösung, behandelt.

Das Mengenverhältnis Reaktionsgemisch : Waschflüssigkeit beträgt in der Regel 1: 0,1 - 1, bevorzugt 1 : 0,2 - 0,8, besonders bevorzugt 1 : 0,3 - 0,7.

Die Wäsche oder Neutralisation kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche oder Neutralisation im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Vorzugsweise werden Siebboden- oder gepackte beziehungsweise Füllkörperkolonnen, Rührbehälter oder Mixer-Settler-Apparate, sowie gepulste Kolonnen oder solche mit rotierenden Einbauten eingesetzt.

Die Vorwäsche e) wird bevorzugt dann eingesetzt, wenn Metallsalze, bevorzugt Kupfer oder Kupfersalze als Inhibitoren (mit)verwendet werden.

Eine Nachwäsche g) kann zur Entfernung von Base- oder Salzspuren aus dem in f) neutralisierten Reaktionsgemisch vorteilhaft sein.

Zur Neutralisation f) kann das gegebenenfalls vorgewaschene Reaktionsgemisch, das noch geringe Mengen an Katalysator und die Hauptmenge an überschüssiger Carbonsäure, beispielsweise (Meth)acrylsäure enthalten kann, mit einer 5 - 25, bevorzugt 5 - 20, besonders bevorzugt 5 - 15 Gew%igen wäßrigen Lösung einer Base, wie beispielsweise Alkali- oder Erdalkalimetalloxide, -hydroxide, -carbonate oder -hydrogencarbonate, bevorzugt Natronlauge, Kalilauge, Natriumhydrogencarbonat, Natriumcarbonat, Kaliumhydrogencarbonat, Kalziumhydroxid, Kalkmilch, Ammoniak, Ammoniakwasser oder Kaliumcarbonat, der gegebenenfalls 5 - 15 Gew% Kochsalz, Kaliumchlorid, Ammoniumchlorid oder

Ammoniumsulfat zugesetzt sein können, besonders bevorzugt mit Natronlauge oder Natronlauge-Kochsalz-Lösung, neutralisiert werden. Der Neutralisationsgrad beträgt bevorzugt 10 bis 80 Mol%, vorzugsweise 20 bis 80 Mol%, besonders bevorzugt 40 bis 80 Mol%, bezogen auf die Säuregruppen enthaltenden Monomere. Diese Neutralisation kann vor und/ oder während der Polymerisation erfolgen, bevorzugt vor der Polymerisation.

Die Zugabe der Base erfolgt in einer Weise, daß die Temperatur im Apparat nicht über 60 °C ansteigt, bevorzugt zwischen 20 und 35 °C beträgt und der pH-Wert 4 - 13 beträgt. Die Abfuhr der Neutralisationswärme erfolgt vorzugsweise durch Kühlung des Behälters mit Hilfe von innenliegenden Kühlschlangen oder über eine Doppelwandkühlung.

Das Mengenverhältnis Reaktionsgemisch : Neutralisationsflüssigkeit beträgt in der Regel 1: 0,1 - 1, bevorzugt 1 : 0,2 - 0,8, besonders bevorzugt 1 : 0,3 - 0,7.

Hinsichtlich der Apparatur gilt das oben Gesagte.

h) Falls ein Lösungsmittel im Reaktionsgemisch enthalten ist, so kann dieses durch Destillation im wesentlichen entfernt werden. Bevorzugt wird gegebenenfalls enthaltenes Lösungsmittel nach Wäsche und/oder Neutralisation aus dem Reaktionsgemisch entfernt, falls gewünscht kann dieses aber auch vor der Wäsche beziehungsweise Neutralisation erfolgen.

Dazu wird das Reaktionsgemisch mit einer derartigen Menge an Lagerstabilisator, bevorzugt Hydrochinonmonomethylether versetzt, daß nach Abtrennung des Lösungsmittels 100 - 500, bevorzugt 200 - 500 und besonders bevorzugt 200 - 400 ppm davon im Zielester (Rückstand) enthalten sind.

Die destillative Abtrennung der Hauptmenge an Lösungsmittel erfolgt beispielsweise in einem Rührkessel mit Doppelwandheizung und/oder innenliegenden Heizschlangen unter vermindertem Druck, beispielsweise bei 20 - 700 mbar, bevorzugt 30 bis 500 und besonders bevorzugt 50 - 150 mbar und einer Temperatur von 40 - 80 °C. Selbstverständlich kann die Destillation auch in einem Fallfilm-oder Dünnschichtverdampfer erfolgen. Dazu wird das Reaktionsgemisch, bevorzugt mehrmals im Kreislauf, unter vermindertem Druck, beispielsweise bei 20 - 700 mbar, bevorzugt 30 bis 500 und besonders bevorzugt 50 - 150 mbar und einer Temperatur von 40 - 80 °C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 - 1, bevorzugt 0,2 - 0,8 und besonders bevorzugt 0,3 - 0,7 m3/m3h, bezogen auf das Volumen des Reaktionsgemisches.

Der Restlösungsmittelgehalt im Rückstand beträgt nach der Destillation in der Regel unter 5 Gew%, bevorzugt 0,5 - 5 % und besonders bevorzugt 1 bis 3 Gew%.

Das abgetrennte Lösungsmittel wird kondensiert und bevorzugt wiederverwendet.

Falls erforderlich kann zusätzlich oder anstelle der Destillation eine Lösungsmittelstrippung i) durchgeführt werden.

Dazu wird der Zielester, der noch geringe Lösungsmittelmengen enthält, auf 50 - 90 °C, bevorzugt 80 - 90 °C erwärmt und die restlichen Lösungsmittelmengen mit einem geeigneten Gas in einer geeigneten Aparatur entfernt. Zur Unterstützung kann gegebenenfalls auch ein Vakuum angelegt werden.

Geeignete Apparaturen sind beispielsweise Kolonnen von an sich bekannter Bauart, die die üblichen Einbauten, z.B. Bödenv, Schüttungen oder gerichtete Packungen, bevorzugt Schüttungen aufweisen. Als Kolonneneinbauten kommen prinzipiell alle gängigen Einbauten in Betracht, beispielsweise Böden, Packungen und/oder Füllkörper. Von den Böden sind Glockenbödenv, Siebböden, Ventilböden, Thormannböden und/oder Dual-Flow-Böden bevorzugtv, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten, bevorzugt.

Denkbar ist hier auch ein Fallfilm-, Dünnfilm- oder Wischfilmverdampfer, wie z.B. ein Luwa-, Rotafilm- oder Sambayverdampfer, der als Spritzschutz beispielsweise mit einem Demister ausgerüstet sein kann.

Geeignete Gase sind unter den Strippbedingungen inerte Gase, bevorzugt sauerstoffhaltige Gase, besonders bevorzugt Luft oder Gemische aus Luft und Stickstoff (Magerluft) oder Wasserdampf, insbesondere solche, die auf 50 bis 100 °C temperiert sind.

Die Strippgasmenge beträgt beispielsweise 5 - 20, besonders. bevorzugt 10 - 20 und ganz besonders bevorzugt 10 bis 15 m3/m3h, bezogen auf das Volumen des Reaktionsgemisches.

Falls notwendig kann der Ester in einem beliebigen Stadium des Aufarbeitungsverfahrens, bevorzugt nach Wäsche/Neutralisation und gegebenenfalls erfolgter Lösungsmittelentfernung einer Filtration j) unterworfen werden, um ausgefallene Spuren an Salzen sowie gegebenenfalls enthaltenem Entfärbungsmittel zu entfernen.

In einer denkbaren Ausführungsform wird die Veresterung a) des Polyalkohols A mit der Carbonsäure B in einem wie oben angeführten molaren Überschuß von mindestens 5:1 in Gegenwart mindestens eines Veresterungskatalysators C und mindestens eines Polymerisationsinhibitors D ohne ein mit Wasser ein Azeotrop bildendes Lösungsmittel durchgeführt.

Die im Überschuß eingesetzte Carbonsäure B wird in einer bevorzugten Ausführungsform im wesentlichen nicht abgetrennt, d.h. es wird lediglich der Anteil an Carbonsäure B aus dem Reaktionsgemisch entfernt, der durch die

Flüchtigkeit bei der angelegten Temperatur bestimmt ist und es werden darüberhinaus keine Maßnahmen zur Abtrennung der Carbonsäure durchgeführt, wie beispielsweise destillative, rektifikative, extraktive, ie.z.B. Wäschen, absorptive, wie z.B. Überleiten über Aktivkohle oder über Ionentauscher, und/oder chemische Schritte, wie z.B. Abfangen der Carbonsäure mit Epoxiden.

Bevorzugt wird die im Reaktionsgemisch enthaltene Carbonsäure B zu nicht mehr als 75 Gew%, besonders bevorzugt zu nicht mehr als 50 Gew%, ganz besonders bevorzugt zu nicht mehr als 25 Gew%, insbesondere zu nicht mehr als 10 Gew% und speziell zu nicht mehr als 5 Gew% aus dem Reaktionsgemisch abgetrennt, bezogen auf die nach Reaktionsende im Reaktionsgemisch enthaltene Carbonsäure B. In einer besonders bevorzugten Ausführungsform kann auf die Stufe b) verzichtet werden, so daß lediglich der Anteil an Reaktionswasser und Carbonsäure B aus dem Reaktionsgemisch entfernt wird, der durch die Flüchtigkeit bei der angelegten Temperatur bestimmt ist. Dies kann bevorzugt durch im wesentlichen vollständige Kondensation verhindert werden.

Weiterhin verbleibt auch der eingesetzte Veresterungskatalysator C im wesentlichen im Reaktionsgemisch.

Das so erhältliche Reaktionsgemisch weist bevorzugt eine Säurezahl gem. DIN EN 3682 von mindestens 25 mg KOH/g Reaktionsgemisch auf, besonders bevorzugt von 25 bis 80 und ganz besonders bevorzugt von 25 bis 50 mg KOH/g auf.

Auf eine Vor- oder Nachwäsche e) oder g) wird bevorzugt verzichtet, lediglich ein Filtrationsschritt j) kann sinnvoll sein.

Anschließend kann das Reaktionsgemisch im Schritt c) verdünnt werden, in diesem Fall wird es bevorzugt innerhalb von 6 Stunden, besonders bevorzugt innerhalb von 3 Stunden zum Hydrogel umgesetzt. Bevorzugt kann es in einem Schritt f) neutralisiert werden.

Die Abfolge der Schritte c), j) und f) ist dabei beliebig.

Die Erfindung betrifft außerdem ein Stoffgemisch enthaltend

- mindestens einen Ester F, erhältlich nach einem der oben beschriebenen Veresterungsverfahren,
- Carbonsäure B und
- Verdünnungsmittel G.

Als weitere Komponenten können enthalten sein

- Veresterungskatalysator C in protonierter oder unprotonierter Form,
- Polymerisationsinhibitor D sowie
- gegebenenfalls Lösungsmittel E, falls ein solches in der Versterung verwendet wurde.

Das Stoffgemisch kann gegebenenfalls neutralisiert sein und einen pH-Wert aufweisen, wie oben unter f) aufgeführt.

Wenn das Stoffgemisch neutralisiert ist, so ist zumindest ein Teil der Carbonsäuren B in deren wasserlöslichen Alkalimetall-, Erdalkalimetall- oder Ammoniumsalze überführt.

Bevorzugtes Stoffgemisch enthält an

- Ester F im Stoffgemisch 0,1 bis 40 Gew.-%, besonders bevorzugt 0,5 bis 20, ganz besonders bevorzugt 1 bis 10, insbesondere 2 bis 5 und speziell 2 bis 4 Gew.-%,
- Carbonsäure B 0,5 - 99,9 Gew%, besonders bevorzugt 0,5 - 50 Gew%, ganz besonders bevorzugt 1 - 25, insbesondere 2 - 15 und speziell 3 bis 5 Gew%,
- Veresterungskatalysator C 0 - 10 Gew%, besonders bevorzugt 0,02
- 5, ganz besonders bevorzugt 0,05 - 2,5 Gew% und insbesondere 0,1 - 1 Gew%,
- Polymerisationsinhibitor D 0 - 5 Gew%, besonders bevorzugt 0,01
- 1,0, ganz besonders bevorzugt 0,02 - 0,75, insbesondere 0,05 - 0,5 und speziell 0,075 - 0,25 Gew%,
- Lösungsmittel E 0 - 10 Gew%, besonders bevorzugt 0 - 5 Gew%, ganz besonders bevorzugt 0,05 - 1,5 Gew% und insbesondere 0,1 - 0,5 Gew%, mit der Maßgabe, daß die Summe immer 100 Gew% beträgt, sowie
- gegebenenfalls Verdünnungsmittel G ad 100 Gew%.

Die nach dem obigen Verfahren erhältlichen Reaktionsgemische und erfindungsgemäßen Stoffgemische können Verwendung finden

- als Radikalvernetzer von wasserabsorbierenden Hydrogelen,
- als Ausgangsstoff für die Herstellung von Polymerdispersionen,
- als Ausgangsstoff für die Herstellung von Polyacrylaten (außer Hydrogelen),
- als Lackrohstoff oder

- als Zementadditiv.

Zur Verwendung als Radikalvernetzer von wasserabsorbierenden Hydrogelen sind insbesondere solche erfindungsgemäßen Stoffgemische geeignet, die eine Wasserlöslichkeit (bei 25 °C in destilliertem Wasser) von mindestens 5 Gew%, bevorzugt mindestens 10 Gew%, besonders bevorzugt mindestens 20 Gew%, ganz besonders bevorzugt mindestens 30 Gew% und insbesondere von mindestens 50 Gew% aufweisen.

k) Das Reaktionsgemisch aus der Veresterung, inklusive deren Aufarbeitungsschritte, soweit diese durchlaufen werden, beispielsweise das Reaktionsgemisch aus f), bzw, wenn auf f) verzichtet wird, aus b), beziehungsweise, wenn auf b) verzichtet wird, das Reaktionsgemisch aus a), kann gegebenenfalls mit zusätzlichen monoethylenisch ungesättigten Verbindungen N versetzt werden, die keine Säuregruppen tragen, aber mit den hydrophilen Monomeren M copolymerisierbar sind, kann dann zur Herstellung von wasserabsorbierenden Hydrogelen in Gegenwart mindestens eines Radikalstarters K und gegebenenfalls mindestens einer Pfropfgrundlage L polymerisiert werden.

Vorteilhaft kann

1) das Reaktionsgemisch aus k) nachvernetzt werden.

Zur Herstellung k) dieser hydrophilen, hochquellfähigen Hydrogele geeignete hydrophile Monomere M sind beispielsweise polymerisationsfähige Säuren, wie Acrylsäure, Methacrylsäure, Ethacrylsäure, $\alpha$-Chloracrylsäure, Crotonsäure, Maleinsäure, Maleinsäureanhydrid, Vinylsulfonsäure, Vinylphosphonsäure, Maleinsäure einschließlich deren Anhydrid, Fumarsäure, Itaconsäure, Citraconsäure, Mesaconsäure, Glutaconsäure, Aconitsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Allylphosphonsäure, Styrolsulfonsäure, 2-Acrylamido-2methylpropansulfonsäure, 2-Acrylamido-2-methylpropanphosphonsäure sowie deren Amide, Hydroxyalkylester und aminogruppen- oder ammoniumgruppenhaltige Ester und Amide. Die Monomeren können allein oder in Mischung untereinander eingesetzt werden. Des weiteren wasserlösliche N-Vinylamide oder auch Diallyldimethyl-ammoniumchlorid. Bevorzugte hydrophile Monomere sind Verbindungen der Formel V

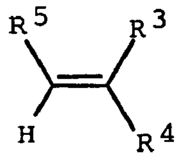

worin

$R^3$ Wasserstoff, Methyl oder Ethyl,

$R^4$ die Gruppe -COOR$^6$, eine Sulfonylgruppe oder Phosphonylgruppe, eine mit einem ($C_1$-$C_4$)-Alkylalkohol veresterte Phosphonyl- gruppe oder eine Gruppe der Formel VI

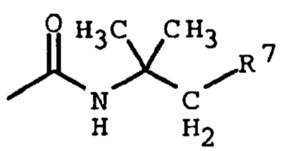

$R^5$ Wasserstoff, Methyl, Ethyl oder eine Carboxylgruppe,

$R^6$ Wasserstoff, Amino oder Hydroxy-($C_1$-$C_4$)-Alkyl und

$R^7$ eine Sulfonylgruppe, eine Phosphonylgruppe oder eine Carboxylgruppe bedeuten.

Beispiele für ($C_1$-$C_4$)- Alkylalkohol sind Methanol, Ethanol, n-Propanol oder n-Butanol.

Besonders bevorzugte hydrophile Monomere sind Acrylsäure und Methacrylsäure.

Zur Optimierung von Eigenschaften kann es sinnvoll sein, zusätzliche monoethylenisch ungesättigte Verbindungen N einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäure, z. B. Acrylamid, Methacrylamid und N-Vinylformamid, N-Vinylacetamid, N-Methyl-vinylacetamid, Acrylnitril und Methacrylnitril. Weitere geeignete Verbindungen sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z. B. Ethylvinylether oder Butylvinylether, Ester von monoethylenisch ungesättigten $C_3$- bis $C_6$-Carbonsäuren, z. B. Ester aus einwertigen $C_1$- bis $C_{18}$-Alkoholen und Acrylsäure, Methacrylsäure oder Maleinsäure, Halbester von Maleinsäure, z. B. Maleinsäuremono-methylester, N-Vinyllactame wie N-Vinylpyrrolidon oder N-Vinylcaprolactam, Acrylsäure- und Methacrylsäureester von alkoxylierten einwertigen, gesättigten Alkoholen, z. B. von Alkoholen mit 10 bis 25 C-Atome, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, z. B. Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-% zugesetzt.

Bevorzugt bestehen die vernetzten (Co)Polymere aus Säuregruppen tragenden monoethylenisch ungesättigten Monomeren, die gegebenenfalls vor oder nach der Polymerisation in ihre Alkali- oder Ammoniumsalze überführt werden, und aus 0 - 40 Gew.-% bezogen auf ihr Gesamtgewicht keine säuregruppentragenden monoethylenisch ungesättigten Monomeren.

Die Herstellung (meth)acrylsäurehaltiger (Co)Polymere, Polyacrylsäuren und Superabsorbern ist vielfach vorbeschrieben und daher hinreichend bekannt, siehe z.B. "Modern Superabsorbent Polymer Technology", F.L. Buchholz and A.T. Graham, Wiley-VCH, 1998.

Bevorzugt sind solche Hydrogele, die durch vernetzende Polymerisation oder Copolymerisation von säuregruppentragenden monoethylenisch ungesättigten Monomeren M oder deren Salzen erhalten werden.

Bei dem Verfahren zur Nachvernetzung wird das Ausgangspolymer mit einem Nachvernetzer behandelt und bevorzugt während oder nach dem Behandeln durch Temperaturerhöhung nachvernetzt und getrocknet, wobei der Vernetzer bevorzugt in einem inerten Lösemittel enthalten ist. Unter inerten Lösemittel werden solche verstanden, die bei der Reaktion weder mit dem Ausgangspolymer noch mit dem Nachvernetzer im wesentlichen reagieren. Bevorzugt sind solche Lösemittel, die zu mehr als 90%, bevorzugt mehr als 95%, besonders bevorzugt mehr als 99%, insbesondere zu mehr als 99,5% nicht chemisch mit dem Ausgangspolymer oder Nachvernetzer reagieren.

Bevorzugt zur Nachvernetzung 1) und Trocknung m) ist dabei der Temperaturbereich zwischen 30 und 250°C, insbesondere 50 - 200 °C, ganz besonders bevorzugt ist der Bereich zwischen 100 - 180°C. Die Aufbringung der Oberflächennachvernetzungslösung erfolgt bevorzugt durch Aufsprühen auf das Polymere in geeigneten Sprühmischern. Im Anschluß an das Aufsprühen wird das Polymerpulver thermisch getrocknet, wobei die Vernetzungsreaktion sowohl vor als auch während der Trocknung stattfinden kann. Bevorzugt ist das Aufsprühen einer Lösung des Vernetzers in Reaktionsmischern oder Misch- und Trocknungsanlagen wie beispielsweise Lödige-Mischer, BEPEX-Mischer, NAUTA-Mischer, SHUGGI-Mischer oder PROCES-SALL. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner wie z.B. ein Hordentrockner, ein Drehrohrofen, oder eine beheizbare Schnecke. Es kann aber auch z.B. eine azeotrope Destillation als Trocknungsverfahren benutzt werden. Die bevorzugte Verweilzeit bei dieser Temperatur im Reaktionsmischer oder Trockner beträgt unter 60 min., besonders bevorzugt unter 30 min.

Bevorzugt sind obige Verfahren, wobei das Ausgangspolymere eine polymere Acrylsäure oder ein Polyacrylat ist, insbesondere eine polymere Acrylsäure oder ein Polyacrylat, die über radikalische Polymerisation erhalten wurden und bei denen ein mehrfunktioneller ethylenisch ungesättigter Radikalvernetzer verwendet wurde.

Bevorzugt werden solche Verfahren in denen das Stoffgemisch enthaltend Radikalvernetzer, also den Ester F, und Verdünnungsmittel G in einem Verhältnis von 0,1 - 20 Gew.-%, insbesondere 0,5 - 10 Gew.-% bezogen auf die Masse des Ausgangspolymeren eingesetzt wird.

Bevorzugt werden solche Verfahren in denen der Radikalvernetzer in einer Dosierung von 0,01 - 5,0 Gew.%, bevorzugt 0,02 - 3,0 Gew.%, ganz besonders bevorzugt 0,03 - 2,5 Gew.%, insbesondere 0,05 - 1,0 und speziell 0,1 bis 0,75 Gew.% bezogen auf das Ausgangspolymer verwendet wird.

Gegenstand der Erfindung sind auch Polymere, hergestellt nach einem der oben genannten Verfahren und deren Verwendung in Hygieneartikeln, Verpackungsmaterialien und in Nonwovens, sowie die Verwendung von einem oben genannten Stoffgemisch zur Herstellung von vernetzten oder durch Wärmebehandlung vernetzungsfähigen Polymeren, insbesondere in Lacken und Farben.

Die dabei einzusetzenden hydrophilen, hochquellfähigen Hydrogele (Ausgangspolymere) sind insbesondere Polymere aus (co)polymerisierten hydrophilen Monomeren M, Pfropf(co)polymere von einem oder mehreren hydrophilen Monomeren M auf eine geeignete Pfropfgrundlage L, vernetzte Cellulose- oder Stärkeether oder in wäßrigen Flüssigkeiten quellbare Naturprodukte, wie beispielsweise Guarderivate. Diese Hydrogele sind dem Fachmann bekannt und beispielsweise beschrieben in US-4 286 082, DE-C-27 06 135, US-4 340 706, DE-C-37 13 601, DE-C-28 40 010, DE-A-43 44 548, DE-A40 20 780, DE-A-40 15 085, DE-A-39 17 846, DE-A-38 07 289, DE-A-35 33 337, DE-A-35 03 458, DE-A-42 44 548, DE-A-42 19 607, DE-A-40 21 847, DE-A-38 31 261, DE-A-35 11 086, DE-A-31 18 172, DE-A-30 28 043, DE-A-44 18 881, EP-A-0 801 483, EP-A-0 455 985, EP-A-0 467 073, EP-A-0 312 952, EP-A-0 205 874, EP-A-0 499 774, DE-A 26 12 846, DE-A-40 20 780 EP-A-0 20 5674, US-5 145 906, EP-A-0 530 438, EP-A-0 670 073, US4 057 521, US-4 062 817, US-4 525 527, US-4 295 987, US-5 011 892, US-4 076 663 oder US-4 931 497. Besonders geeignet sind auch hochquellfähige Hydrogele aus einem Herstellprozeß wie in WO 01/38402 beschrieben, sowie anorganischorganische hybride hochquellfähige Hydrogele wie in DE 198 54 575 beschrieben. Der Inhalt der vorstehend genannten Patentdokumente, insbesondere die nach den Verfahren hergestellten Hydrogele, sind ausdrücklich Bestandteil der vorliegenden Offenbarung.

Geeignete Pfropfgrundlagen L für hydrophile Hydrogele, die durch Pfropfcopolymerisation olefinisch ungesättigter Säuren erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Das wasserabsorbierende Polymer kann über radikalische Pfropfcopolymerisation von Acrylsäure oder Acrylat auf eine wasserlösliche Polymermatrix erhalten werden. Geeignete wasserlösliche Polymermatrices sind beispielsweise, aber nicht ausschliesslich, Alginate, Polyvinylalkohol, und Polysacharide wie etwa Stärke. Bei der Pfropfcopolymerisation im erfindungsgemäßen Sinne wird ein mehrfunktioneller ethylenisch ungesättigter Radikalvernetzer eingesetzt.

Das wasserabsorbierende Polymer kann ein organisch-anorganisches Hybridpolymer aus einer polymeren Acrylsäure oder einem Polyacrylat einerseits und einem Silikat, Aluminat, oder Alumosilikat andererseits sein. Insbesondere können polymere Acrylsäure oder Polyacrylat verwendet werden, die über radikalische Polymerisation erhalten wurden, und bei denen ein mehrfunktioneller ethylenisch ungesättigter Radikalvernetzer verwendet wurde und bei deren Herstellprozeß ein in Wasser lösliches Silikat oder lösliches Aluminat oder Gemische von beiden eingesetzt wurde.

Bevorzugte Hydrogele sind insbesondere Polyacrylate, Polymethacrylate sowie die in US-4 931 497, US-5 011 892 und US-5 041 496 beschriebene Pfropfpolymere. Ganz besonders bevorzugte Hydrogele sind die in WO 01/38402 beschriebenen Kneterpolymere und die in DE 198 545 75 beschriebenen hybriden organisch-anorganischen Hydrogele auf Basis von Polyacrylaten.

Die erfindungsgemäß hergestellten, als Radikalvernetzer in Hydrogelen verwendbaren Substanzen können allein oder in Kombination mit anderen Vernetzern, beispielsweise Innen- oder Oberflächenvernetzern verwendet werden, beispielsweise den folgenden:

Geeignete Vernetzer sind insbesondere Methylenbisacryl- bzw. - methacrylamid, Ester ungesättigter Mono- oder Polycarbonsäuren von Polyolen, wie Diacrylat oder Triacrylat, z. B. Butandiol-oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Besonders bevorzugt werden im erfindungsgemäßen Verfahren jedoch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Monoethylenglykol-diallylether, Glyceroldi- und Triallylether, Polyallylether auf Basis Sorbitol, sowie ethoxylierte Varianten davon. Weiterhin besonders bevorzugte Vernetzer sind die Polyethylenglykol-diacrylate, ethoxylierte Derivate von Trimethylolpropantriacrylat z.B. Sartomer SR 9035, sowie ethoxylierte Derivate von Glycerindiacrylat und Glycerintriacrylat. Selbstverständlich können auch Gemische der obigen Vernetzer eingesetzt werden.

Ganz besonders bevorzugt sind solche Hydrogele, die mit einem erfindungsgemäß hergestellten Ester F als Radikalvernetzer hergestellt werden.

Das wasserabsorbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere enthalten (0,001 - 10 mol-%), ganz besonders bevorzugt sind jedoch Polymere, die über radikalische Polymerisation erhalten wurden und bei denen ein mehrfunktioneller ethylenisch ungesättigter Radikalvernetzer verwendet wurde.

Die hydrophilen, hochquellfähigen Hydrogele können durch an sich bekannte Polymerisationsverfahren hergestellt werden. Bevorzugt ist die Polymerisation in wäßriger Lösung nach dem Verfahren der sogenannten Gelpolymerisation. Dabei werden wie oben angeführt verdünnte, bevorzugt wäßrige, besonders bevorzugt 15 bis 50 Gew.-% ige wäßrige Lösungen eines oder mehrerer hydrophiler Monomerer und gegebenenfalls einer geeigneten Pfropfgrundlage L in Gegenwart eines Radikalinitiators bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0°C und 150°C, vorzugsweise zwischen 10°C und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. Zur Auslösung der Polymerisation können energiereiche elektromagnetische Strahlen oder die üblichen chemischen Polymerisationsinitiatoren K herangezogen werden, z. B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie $(NH_4)_2S_2O_8$, $K_2S_2O_8$ oder $H_2O_2$.

Sie können gegebenenfalls in Kombination mit Reduktionsmitteln wie z.B. Ascorbinsäure, Natriumhydrogensulfit, und Eisen(11)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie z. B. Mannichaddukte aus Sulfinsäuren, Aldehyden und Aminoverbindungen, wie sie in der DE-C-1 301 566 beschrieben sind, verwendet werden. Durch mehrstündiges Nachheizen der Polymerisatgele im Temperaturbereich 50° bis 130°C, vorzugsweise 70° bis 100°C, können die Qualitätseigenschaften der Polymerisate noch verbessert werden.

Die erhaltenen Gele werden zu 0 - 100 mol-%, bevorzugt zu 25 - 100 mol-%, und besonders bevorzugt zu 50 - 85 mol-% bezogen auf eingesetztes Monomer neutralisiert, wobei die üblichen Neutralisationsmittel verwendet werden können, bevorzugt Alkalimetallhydroxide, Alkalimetalloxide oder die entsprechenden Alkalimetallcarbonate, besonders bevorzugt jedoch Natriumhydroxid, Natriumcarbonat und Natriumhydrogencarbonat.

Üblicherweise wird die Neutralisation durch Einmischung des Neutralisationsmittels als wäßrige Lösung oder bevorzugt auch als Feststoff erreicht. Das Gel wird hierzu mechanisch zerkleinert, z.B. mittels eines Fleischwolfes, und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt.

Die Polymerisation an sich kann aber auch nach jedem anderen der in der Literatur beschriebenen Verfahren durchgeführt werden. Insbesondere kann die Neutralisation der Acrylsäure auch vor der Polymerisation durchgeführt werden, wie oben im Schritt f) beschrieben. Die Polymerisation kann dann in einem dem Fachmann bekannten Bandreaktor oder einem Knetreaktor kontinuierlich oder auch diskontinuierlich durchgeführt werden. Bei Durchführung der Polymerisation in einem Bandreaktor ist die Initiation mittels elektromagnetischer Strahlung, bevorzugt mittels W-Strahlung, oder alternativ die Initiation mit einem Redoxinitiatorsystem besonders bevorzugt. Ganz besonders bevorzugt ist auch die Kombination beider Initiationsmethoden: elektromagnetische Strahlung und chemisches Redoxinitiatorsystem simultan.

n) Das getrocknete Hydrogel kann hiernach gemahlen und gesiebt werden, wobei zur Mahlung üblicherweise Walzenstühle, Stiftmühlen oder Schwingmühlen eingesetzt werden können. Die bevorzugte Partikelgröße des gesiebten Hydrogels liegt vorzugsweise im Bereich 45 - 1000 $\mu$m, bevorzugt bei 45 - 850 $\mu$m, besonders bevorzugt bei 200 - 850 $\mu$m, und ganz besonders bevorzugt bei 300 - 850 $\mu$m. In diesen Bereichen liegen bevorzugt 80 Gew.-% der Teilchen, insbesondere 90 Gew.-% der Teilchen. Die Größenverteilung kann mit etablierten Lasermethoden bestimmt werden.

Gegenstand der vorliegenden Erfindung sind weiterhin vernetzte Hydrogele, die mindestens ein hydrophiles Monomer M in einpolymerisierter Form enthalten und vernetzt sind mit einem Ester F eines Polyalkohols A mit mindestens einer ethylenisch ungesättigten Carbonsäure B. Der Ester kann erfindungsgemäß oder auf eine im Stand der Technik bekannte Weise hergestellt werden, bevorzugt auf erfindungsgemäße Weise.

Als Ester F sind solche Verbindungen einsetzbar, wie sie vorstehend beschrieben sind. Polyalkohole A und ethylenisch ungesättigten Carbonsäuren B sind ebenfalls solche, wie vorstehend beschrieben.

Bevorzugt werden als Ester F solche eingesetzt, bei denen das Polyalkohol A ausgewählt ist aus der Liste Polyol, das als zusätzliche Funktionalität mindestens eine Ether-, Carboxyl- oder $C_1$ - $C_4$-Alkyloxycarbonylfunktion aufweist, Zuckeralkohole, teilweise alkoxylierte Zuckeralkohole, Polyesterole, zumindest teilweise alkoxylierte Polyesterole und zumindest teilweise verseifte, alkoxylierte Polyesterole, wie sie jeweils oben beschrieben sind.

Besonders bevorzugt sind solche Ester F, bei denen das Polyalkohol A ausgewählt ist aus der Liste Ditrimethylolpropan, Dipentaerythrit, Dimethylolpropionsäure und Dimethylolbuttersäure.

Weitere bevorzugte Ester F sind solche der Formel VII, wie oben definiert, in denen y unabhängig voneinander

- für x=2 eine Zahl größer als 8, bevorzugt mehr als 10, besonders bevorzugt mehr als 12 und insbesondere mindestens 15 und
- für x=3 oder größer eine Zahl größer als 7, bevorzugt mehr als 9, besonders bevorzugt mehr als 12 und insbesondere mindestens 15 ist.

Gleichfalls bevorzugt sind Ester F der Formel VII, wie oben definiert, in denen y unabhängig voneinander bis zu 6, besonders bevorzugt bis zu 4 und ganz besonders bevorzugt 4 bedeutet.

Verwendbar sind auch Ester F der Formel VII, wie oben definiert, in denen für x = 2 y Werte von 0, 1 oder 2 und für x = 3 y Werte von 0 oder 1 annehmen kann.

Die durch die Formel VII beschriebenen Polyalkohole A in den Estern F, die als Vernetzer in den vorstehend genannten Hydrogelen verwendet werden, können jeweils ethoxyliert, propoxyliert oder gemischt ethoxyliert und propoxyliert und insbesondere ausschließlich ethoxyliert sein, d.h. $R^{10}$ in Formel VII kann beispielsweise unabhängig voneinander Wasserstoff und/oder Methyl und insbesondere ausschließlich Wasserstoff bedeuten.

Besonders bevorzugt sind als Ester F der Formel VII solche Ester F eines Polyalkohols A mit mindestens einer ethylenisch ungesättigten Carbonsäure B, wobei es sich bei dem Polyalkohol A um ein pro Glycerin drei bis vierfach ethoxyliertes Glycerin oder um ein pro Hydroxygruppe vierfach ethoxyliertes Trimethylolpropan oder Pentaerythrit handelt.

Der CRC-Wert [g/g] der erfindungsgemäßen Hydrogel-formenden Polymere kann nach den in der Beschreibung angegebenen Methoden gemessen werden und liegt bevorzugt größer 15, insbesondere 16, 18, 20 ,22, 24, oder höher, besonders bevorzugt bei 25 insbesondere bei 26, 27, 28, 29, insbesondere bevorzugt bei 30, 31, 32, 33, 34, 35, 36, 37 oder höher.

Der AUL-0,7psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Der AUL-0,5psi-Wert [g/g] der erfindungsgemäßen Hydrogel-formenden Polymere kann nach den in der Beschreibung angegebenen Methoden gemessen werden und liegt bevorzugt größer 8, insbesondere 9, 10, 11, 12, 13, 14 oder höher, besonders bevorzugt bei 15 insbesondere bei 16, 17, 18, 19, oder höher, insbesondere bevorzugt größer 20, insbesondere 21, 22, 23, 24, 25, 26, 27, 28, oder höher.

Einsatz und Verwendung der erfindungsgemäßen Hydrogel-formenden Polymere

Die vorliegende Erfindung betrifft ferner die Verwendung der oben genannten Hydrogel-formenden Polymeren in Hygieneartikel, umfassend

(P) eine obere flüssigkeitsdurchlässige Abdeckung

(Q) eine untere flüssigkeitsundurchlässige Schicht

(R) einen zwischen (P) und (Q) befindlichen Kern, enthaltend

10 - 100 Gew.-% des erfindungsgemäßem Hydrogel-formenden Polymer
0 - 90 Gew.-% hydrophiles Fasermaterial

bevorzugt 20 - 100 Gew.-% des erfindungsgemäßem Hydrogel-formenden Polymer, 0 - 80 Gew.-% hydrophiles Fasermaterial
mehr bevorzugt 30 - 100 Gew.-% des erfindungsgemäßem Hydrogel-formenden Polymer, 0 - 70 Gew.-% hydrophiles Fasermaterial
noch mehr bevorzugt 40 - 100 Gew.-% des erfindungsgemäßem Hydrogel-formenden Polymer, 0 - 60 Gew.-% hydrophiles Fasermaterial
besser bevorzugt 50 - 100 Gew.-% des erfindungsgemäßem Hydrogel-formenden Polymer 0 - 50 Gew.-% hydrophiles Fasermaterial
besonders bevorzugt 60 - 100 Gew.-% des erfindungsgemäßem Hydrogel-formenden Polymer, 0 - 40 Gew.-% hydrophiles Fasermaterial
insbesondere bevorzugt 70 - 100 Gew.-% des erfindungsgemäßem Hydrogel-formenden Polymer, 0 - 30 Gew.-% hydrophiles Fasermaterial
außerordentlich bevorzugt 80 - 100 Gew.-% des erfindungsgemäßem Hydrogel-formenden Polymer, 0 - 20 Gew.-% hydrophiles Fasermaterial
am meisten bevorzugt 90 - 100 Gew.-% des erfindungsgemäßem Hydrogel-formenden Polymer, 0 - 10 Gew.-% hydrophiles Fasermaterial

(S) gegebenenfalls eine sich unmittelbar oberhalb und unterhalb des Kerns (R) sich befindende Tissueschicht und

(T) gegebenenfalls eine zwischen (P) und (R) sich befindende Aufnahmeschicht.

Die Prozentangaben sind so zu verstehen, dass bei 10 - 100 Gew.%, 11, 12, 13, 14, 15, 16, 17, 18, 19 bis jeweils 100 Gew.-% an erfindungsgemäßem Hydrogel-formenden Polymer und alle dazwischenliegendnen %-Angaben

(z.B. 12,2%) möglich sind und entsprechend hydrophiles Fasermaterial von 0 bis jeweils 89, 88, 87, 86, 85, 83, 82, 81 Gew.-% und dazwischen liegende Prozentangaben (z.B. 87,8%) möglich sind. Liegen weitere Materialien im Kern vor, so verringern sich entsprechend die Prozentwerte an Polymer und Faser. Das ananloge gilt für die bevorzugten Bereiche, so z.B. bei außerordentlich bevorzugt können 81, 82, 83, 84, 85, 86, 87, 88, 89 Gew.% für das erfindungsgemäßem Hydrogel-formenden Polymer und entsprechend 19, 18, 17, 16, 15, 14, 13, 12, 11 Gew-% des Fasermaterials vorliegen. So können im bevorzugten Bereich 20, 21, 22, 23, 24, 25, 26, 27, 28, 29 bis 100 Gew.-% erfindungsgemäßes Hydrogel-formendes Polymer, im mehr bevorzugten Bereich 30, 31, 32, 33, 34, 35, 36, 37, 38, 39 bis 100 Gew.-% erfindungsgemäßes Hydrogel-formendes Polymer, im noch mehr bevorzugten Bereich 40, 41, 42, 43, 44, 45, 46, 47, 48, 49 bis 100 Gew.-% erfindungsgemäßes Hydrogel-formendes Polymer, im besser bevorzugten Bereich 50, 51, 52, 53, 54, 55, 56, 57, 58, 59 bis 100 Gew.-% erfindungsgemäßes Hydrogel-formendes Polymer, im besonders bevorzugten Bereich 60, 61, 62, 63, 64, 65, 66, 67, 68, 69 bis 100 Gew.-% erfindungsgemäßes Hydrogel-formendes Polymer, im insbesonders bevorzugten Bereich 70, 71, 71, 72, 73, 74, 75, 76, 77, 78, 79 bis 100 Gew.-% erfindungsgemäßes Hydrogel-formendes Polymer und im am meisten bevorzugten Bereich 90, 91, 92, 93, 94, 95, 96, 97, 98, 99 oder 100 Gew.-% erfindungsgemäßes Hydrogel-formendes Polymer vorliegen.

Unter Hygieneartikel sind dabei sowohl Inkontinenzeinlagen und Inkontinenzhosen für Erwachsene als auch Windeln für Babys zu verstehen.

Bei der flüssigkeitsdurchlässigen Abdeckung (P) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355 A1, EP 102 388 3 A2.

Die flüssigkeitsundurchlässige Schicht (Q) besteht in der Regel aus einer Folie aus Polyethylen oder Polypropylen.

Der Kern (R) enthält neben dem erfindungsgemäßen Hydrogel-formendem Polymer hydrophiles Fasermaterial. Unter hydrophil ist zu verstehen, daß sich wäßrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 - 200 $\mu$m, bevorzugt 10 - 100 $\mu$m. Darüberhinaus haben die Fasern eine Mindestlänge von 1 mm.

Der Aufbau und die Form von Windeln ist allgemein bekannt und beispielsweise in der WO 95 / 26 209 S. 66 Zeile 34 bis S. 69 Zeile 11, DE 196 04 601 A1, EP-A-0 316 518 und EP-A-0 202 127 beschrieben. Allgemein werden Windeln und andere Hygieneartikel auch in WO 00/65084, insbesondere auf Seiten 6-15, WO 00/65348, insbesondere auf Seiten 4 - 17, WO 00/35502, insbesondere Seiten 3-9, DE 19737434, WO 98/8439 beschrieben. Hygieneartikel für die Damenhygiene werden in folgenden Literaturstellen beschrieben. Die erfindungsgemäßen wäßrige Flüssigkeiten absorbierende Hydrogel-formende Polymere können dort eingestzt werden. Literaturstellen Damenhygiene: WO 95/24173: Absorption Article for Controlling Odour, WO 91/11977: Body Fluid Odour Control, EP 389023: Absorbent Sanitary Articles, WO 94/25077: Odour Control Material, WO 97/01317: Absorbent Hygienic Article, WO 99/18905, EP 834297, US 5,762,644, US 5,895,381, WO 98/57609, WO 2000/065083, WO 2000/069485, WO 2000/069484, WO 2000/069481, US 6,123,693, EP 1104666, WO 2001/024755, WO 2001/000115, EP 105373, WO 2001/041692, EP 1074233. Tampons werden in folgenden Schriften beschrieben: WO 98/48753, WO 98/41179, WO 97/09022, WO 98/46182, WO 98/46181, WO 2001/043679, WO 2001/043680, WO 2000/061052, EP 1108408, WO 2001/033962, DE 200020662, WO 2001/001910, WO 2001/001908, WO 2001/001909, WO 2001/001906, WO 2001/001905, WO 2001/24729. Inkontinenzartikel werden in folgenden Schriften beschrieben: Disposable Absorbent Article for Incontinent Individuals: EP 311344 Beschreibung S. 3 - 9; Disposable Absorbent Article: EP 850623; Absorbent Article: WO 95/26207; Absorbent Article: EP 894502; Dry Laid Fibrous Structure: EP 850 616; WO 98/22063; WO 97/49365; EP 903134; EP 887060; EP 887059; EP 887058; EP 887057; EP 887056; EP 931530; WO 99/25284; WO 98/48753. Damenhygiene- und Inkontinenzartikel wird in folgenden Schriften beschrieben: Catamenial Device: WO 93/22998 Beschreibung S. 26 - 33; Absorbent Members for Body Fluids: WO 95/26209 Beschreibung S. 36 - 69; Disposable Absorbent Article: WO 98/20916 Beschreibung S. 13 - 24; Improved Composite Absorbent Structures: EP 306262 Beschreibung S. 3 - 14; Body Waste Absorbent Article: WO 99/45973. Diese Referenzen und die Referenzen dort, werden hiermit ausdrücklich in die Offenbarung der Erfindung einbezogen.

Die erfindungsgemäßen Hydrogel-formenden Polymere eignen sich hervorragend als Absorptionsmittel für Wasser und wäßrige Flüssigkeiten, so daß sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Einlagerung und Fixierung der erfindungsgemäßen hochquellfähigen Hydrogele

Zusätzlich zu den oben beschriebenen hochquellfähigen Hydrogelen liegen in der absorbierenden Zusammensetzung gemäß der vorliegenden Erfindung Kompositionen vor, welche die hochquellfähigen Hydrogele enthalten oder an denen sie fixiert sind. Jede Komposition ist geeignet, die die hochquellfähigen Hydrogele aufnehmen kann und die darüber hinaus in die Absorptionsschicht integriert werden kann. Eine Vielzahl derartiger Zusammensetzungen ist bereits bekannt und eingehend in der Literatur beschrieben. Eine Komposition zum Einbau der hochquellfähigen Hydrogele kann z. B. eine Fasermatrix sein, die aus einem Cellulosefasergemisch (air-laid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozeß eines air-laid web ist beispielsweise geschildert in WO 98/28 478. Des weiteren können offenporige Schäume oder ähnliches zum Einbau hochquellfähiger Hydrogele dienen.

Alternativ kann eine derartige Komposition durch Fusion zweier Einzelschichten entstehen, wobei eine oder besser eine Vielzahl an Kammern gebildet werden, die die hochquellfähigen Hydrogele enthalten. Ein derartiges Kammersystem ist detailliert geschildert in EP 0 615 736 A1 S. 7 Zeile 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die hochquellfähigen Hydrogelpartikel zurückzuhalten. Obige Beispiele zur Komposition der absorbierenden Zusammensetzung schließen auch Laminate aus mindestens zwei Schichten mit ein, zwischen die die hochquellfähigen Hydrogele eingebaut und fixiert werden.

Generell besteht die Möglichkeit, Hydrogelpartikel innerhalb des Absorbent Cores zur Verbesserung der sog. Dry- und Wet-Integrity zu fixieren. Unter Dry- und Wet-Integrity versteht man die Fähigkeit, hochquellfähige Hydrogele derart in die absorbierende Zusammensetzung einzubauen, daß sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in WO 95/26 209 S. 37 Zeile 36 bis S. 41 Zeile 14. Besagte Passage ist somit Bestandteil dieser Erfindung. Methoden zur Erhöhung der Wet Strength finden sich auch in WO 2000/36216 A1.

Des weiteren kann die absorbierende Zusammensetzung aus einem Trägermaterial, wie z. B. einem Polymerfilm bestehen, auf dem die hochquellfähigen Hydrogelpartikel fixiert werden. Die Fixierung kann sowohl ein- als auch beidseitig vorgenommen werden. Das Trägermaterial kann wasserdurchlässig oder wasserundurchlässig sein.

In obige Kompositionen der absorbierenden Zusammensetzung werden die hochquellfähigen Hydrogele mit einem Gewichtsanteil von 10 bis 100 Gew%, bevorzugt 20 - 100 Gew.-%, mehr bevorzugt 30 - 100 Gew.-%, noch mehr bevorzugt 40 - 100 Gew.-%, besser bevorzugt 50 - 100 Gew.-%, besonders bevorzugt 60 - 100 Gew.-%, insbesondere bevorzugt 70 - 100 Gew.-%, außerordentlich bevorzugt 80 - 100 Gew.-% und am meisten bevorzugt 90 - 100 Gew.-% basierend auf dem Gesamtgewicht der Komposition und der hochquellfähigen Hydrogele eingebaut.

Fasermaterialien der absorbierenden Zusammensetzung

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt Patent WO 95/26 209 S. 28 Zeile 9 bis S. 36 Zeile 8. Besagte Passage ist somit Bestandteil dieser Erfindung.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON®, Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX®), Polypropylenfasern und Polyethylen-Polypropylen-Zweikomponentenfasern, Polyesterfasern, wie

Polyethylenterephthalatfasern (DACRON® oder KO-DEL®), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt zwischen 75°C und 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0,1 bis 100 Denier (Gramm pro 9 000 Meter) bevorzugt verwendet werden. Bevorzugte thermoplastische Fasern weisen eine Länge von 3 bis 50 mm, besonders bevorzugte eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner aus 90 ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird. Bevorzugt wird hydrophiles Fasermaterial eingesetzt. Besonders bevorzugt gelangt Fasermaterial zum Einsatz, das zur Körperseite hin schwach hydrophil und in der Region um die hochquellfähigen Hydrogele am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie z. B. Polyethylenterephthalat (DACRON®), und hydrophiles Nylon (HYDROFIL®). Geeignete hydrophile Fasern können auch erhalten werden durch Hydrophilierung hydrophober Fasern, wie z.B. die Behandlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die hochquellfähigen Hydrogelpartikel werden in das beschriebene Fasermaterial eingebettet. Dies kann auf vielfältige Weise geschehen, indem man z. B. mit dem Hydrogelmaterial und den Fasern zusammen eine Absorptionsschicht in Form einer Matrix aufbaut, oder durch Einlagerung hochquellfähiger Hydrogele in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser: (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene® 557 H, Hercoles, Inc. Wilmington Delaware, USA), Polyacrylamid- Überzüge (beschrieben in U.S. Patent 3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_B$ Monoaldehyde mit saurer Funktionalität, und

insbesondere $C_2$-$C_9$ Polycarbonsäuren. Spezifische Substanzen aus dieser Reihe sind beispielswiese Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, U.S. Patent 3,224,926, U.S. Patent 3,440,135, U.S. Patent 3,932,209, U.S. Patent 4,035,147, U.S. Patent 4,822,453, U.S. Patent 4,888,093, U.S. Patent 4,898,642 und U.S. Patent 5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Herstellungsverfahren der absorbierenden Zusammensetzung

Die absorbierende Zusammensetzung setzt sich zusammen aus Kompositionen, welche hochquellfähige Hydrogele enthalten, und den hochquellfähigen Hydrogelen, die in besagten Kompositionen vorliegen oder daran fixiert sind.

Beispiele für Verfahren, mit denen man eine absorbierende Zusammensetzung erhält, die beispielsweise aus einem Trägermaterial bestehen, an den ein- oder beidseitig hochquellfähige Hydrogele fixiert sind, sind bekannt und von der Erfindung mit eingeschlossen, jedoch nicht limitiert darauf.

Beispiele für Verfahren, mit denen man eine absorbierende Zusammensetzung erhält, die beispielsweise aus in ein Fasermaterial-Gemisch aus synthetischen Fasern (a) und Cellulosefasern (b) eingebetteten hochquellfähigen Hydrogelen (c) besteht, wobei das Mischungsverhältnis von (100 bis 0) synthetische Faser : (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so daß eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0,5 Sekunde bis 3 Minuten, bevorzugt 1 Sekunde bis 1 Minute.

Die absorbierende Zusammensetzung wird im allgemeinen beispielsweise mit einer für Flüssigkeit durchlässien Deckschicht und einer für Flüssigkeit undurchlässigen Unterschicht versehen. Wieterhin werden Beinabschlüsse und Klebebänder angebracht und so der Hygieneartikel fertiggestellt. Die Materialien und Arten der durchlässien Deckschicht und undurchlässigen Unterschicht, sowie der Beinabschlüsse und Klebebänder sind dem Fachmann bekannt und nicht besonders eingeschränkt. Beispiele hierfür finden sich in WO 95/26 209.

Der Vorteil der vorliegenden Erfindung beruht darin, daß die als Vernetzer verwendbaren Ester F nach ihrer Herstellung nicht aufgereinigt werden müssen, besonders daß nicht die Carbonsäure B, beispielsweise Acrylsäure, abgetrennt werden muß, da diese in der Regel ein Monomer zur Herstellung der Hydrogele darstellt.

Experimenteller Teil

In dieser Schrift verwendete ppm- und Prozentangaben beziehen sich, falls nicht anders angegeben, auf Gewichtsprozente und - ppm.

Das erfindungsgemäße Verfahren wird durch das nachfolgende Beispiel näher erläutert.

Beispiele

Herstellung von Acrylat-Rohestern als Superabsorbervernetzer

Die Herstellung der Superabsorbervernetzer erfolgt in den Beispielen durch Veresterung von Polyetherolen mit Acrylsäure wobei die Abtrennen des Wassers in einer azeotropen Destillation erfolgt. Veresterungskatalysator ist in den Beispielen Schwefelsäure. Die Reaktanden werden zusammen mit einer Stabilisatormischung bestehend aus Hydrochinonmonomethylether, Triphenylphosphit und Hypophosphorige Säure in den Beispielen in Methylcyclohexan als Schleppmittel vorgelegt. Die Reaktionsmischung wird dann auf ca. 98°C erwärmt bis die azeotrope Destillation beginnt. Während der azeotropen Destillation steigt die Temperatur in der Reaktionsmischung an. Die abgetrennte Wassermenge wird bestimmt. Die Destillation wird abgebrochen, wenn mindestens die theoretische Wassermenge abgetrennt wurde. Anschließend wird das Schleppmittel in einer Vakuumdestillation entfernt. Das Produkt wird abgekühlt und als Vernetzer in der Superabsorberherstellung eingesetzt.

Umsatz und Ausbeute der Umsetzung wird nicht genau bestimmt, da das in der Veresterung abgetrennte Wasser auch Acrylsäure enthält und auch während der Vakuumdestillation des Schleppmittels Acrylsäure entfernt wird. Auch der Rohester enthält noch freie Acrylsäure, die zusammen mit dem Katalysator titriert wird (Säurezahl).

Alle Mengenangaben sind, soweit nicht anders angegeben, Gewichtsteile.

Herstellung des Esters

Säurezahlen wurden gem. DIN EN 3682 bestimmt.

Beispiel 1 (Polyethylenglycol 400 Diacrylat)

740 Teile Polyethylenglycol mit einer mittleren Molmasse von ca. 400 (Pluriol 400®, BASF AG) wurden mit 320 Teilen Acrylsäure und 5 Teilen Schwefelsäure in 354 Teilen Methylcyclohexan verestert. Als Hilfsstoffe wurden 3 Teile Hydrochinonmonomethylether, 1 Teil Triphenylphosphit und 1 Teil Hypophosphoriger Säure zugesetzt. Es wurden 65 Teile Wasser abgetrennt bevor das Schleppmittel durch Vakuumdestillation entfernt wurde. Das Produkt wurde über K300-Filter gereinigt. Die Säurezahl betrug 50,4 mg KOH/g und wurde durch Zugabe von 72 Teilen Acrylsäure auf Säurezahl 99 mg KOH/g eingestellt. Die Viskosität des leicht gelblichen Produktes (Jodfarbzahl 1) betrug 91 mPas.

Beispiel 2 (Polyethylenglycol 600 Diacrylat)

810 Teile Polyethylenglycol mit einer mittleren Molmasse von ca. 600 (Pluriol® 600, BASF AG) wurden mit 234 Teilen Acrylsäure und 5 Teilen Schwefelsäure in 448 Teilen Methylcyclohexan verestert. Als Hilfsstoffe wurden 3 Teilen Hydrochinonmonomethylether, 1 Teil Triphenylphosphit und 1 Teil Hypophosphoriger Säure zugesetzt. Es wurden 40 Teile Wasser abgetrennt bevor das Schleppmittel durch Vakuumdestillation entfernt wurde. Das Produkt wurde über K300-Filter gereinigt. Die Säurezahl betrug 54 mg KOH/g und wurde durch Zugabe von 70 Teilen Acrylsäure auf Säurezahl 100 mg KOH/g eingestellt. Die Viskosität des leicht gelblichen Produktes betrug 100 mPas.

Beispiel 3 (ca. 3fach d.h. einfach je Hydroxygruppe ethoxyliertes TMP Triacrylat)

579 Teile 3fach ethoxyliertes Trimethylolpropan werden mit 562 Teilen Acrylsäure und 5 Teilen Schwefelsäure in 380 Teilen Methylcyclohexan verestert. Als Hilfsstoffe werden 3 Teilen Hydrochinonmonomethylether, 1 Teil Triphenylphosphit und 1 Teil Hypophosphoriger Säure zugesetzt. Es werden 125 Teile Wasser abgetrennt bevor das Schleppmittel durch Vakuumdestillation entfernt wird. Das Produkt wird über K300-Filter gereinigt. Die Säurezahl liegt bei 38 mg KOH/g. Die Viskosität des fast farblosen Produktes (Jodfarbzahl 0-1) liegt bei 200 mPas.

Beispiel 4 (ca. 7fach je Molekül TMP ethoxyliertes TMP Triacrylat)

681 Teile 7fach ethoxyliertes Trimethylolpropan (Polyol TP 70®, Perstorp) wurden mit 414 Teilen Acrylsäure und 5 Teilen Schwefelsäure in 365 Teilen Methylcyclohexan verestert. Als Hilfsstoffe wurden 3 Teilen Hydrochinonmonomethylether, 1 Teil Triphenylphosphit und 1 Teil Hypophosphoriger Säure zugesetzt. Es wurden 102 Teile Wasser abgetrennt bevor das Schleppmittel durch Vakuumdestillation entfernt wurde. Das Produkt wurde über K300-Filter gereinigt. Die Säurezahl betrug 26 mg KOH/g und wurde durch Zugabe von 105 Teilen Acrylsäure auf Säurezahl 99 mg KOH/g eingestellt. Die Viskosität des fast farblosen Produktes (Jodfarbzahl 0-1) betrug 73,2 mPas.

Beispiel 5 (ca. 15fach je Molekül TMP ethoxyliertes TMP Triacrylät)

750 Teile 15fach ethoxyliertes Trimethylolpropan (Emulan® TE15, BASF AG) wurden mit 216 Teilen Acrylsäure und 5 Teilen Schwefelsäure in 322 Teilen Methylcyclohexan verestert. Als Hilfsstoffe wurden 3 Teilen Hydrochinonmonomethylether, 1 Teil Triphenylphosphit und 1 Teil Hypophosphoriger Säure zugesetzt. Es wurden 44 Teile Wasser abgetrennt bevor das Schleppmittel durch Vakuumdestillation entfernt wurde. Das Produkt wurde über K300-Filter gereinigt. Die Säurezahl betrug 36 mg KOH/g. Die Viskosität des fast farblosen Produktes (Jodfarbzahl 0-1) betrug 324 mPas.

Beispiel 6 (ca. 20fach je Molekül TMP ethoxyliertes TMP Triacrylat)

830 Teile ca. 20fach ethoxyliertes Trimethylolpropan wurden mit 216 Teilen Acrylsäure und 5 Teilen Schwefelsäure in 345 Teilen Methylcyclohexan verestert. Als Hilfsstoffe wurden 3 Teilen Hydrochinonmonomethylether, 1 Teil Triphenylphosphit und 1 Teil Hypophosphoriger Säure zugesetzt. Es wurden 44 Teile Wasser abgetrennt bevor das Schleppmittel durch Vakuumdestillation entfernt wurde. Das Produkt wurde über K300-Filter gereinigt. Die Säurezahl betrug 36 mg KOH/g und wurde durch Zugabe von 96 Teilen Acrylsäure auf Säurezahl 101 mg KOH/g eingestellt. Die Viskosität des fast farblosen Produktes (Jodfarbzahl 0-1) betrug 324 mPas.

Beispiel 7 (ca. 3fach je Glycerinmolekül ethoxyliertes Glycerin Triacrylat)

561 Teile ca. 3fach ethoxyliertes Glycerin wird mit 605 Teilen Acrylsäure und 5 Teilen Schwefelsäure in 390 Teilen Methylcyclohexan verestert. Als Hilfsstoffe werden 3 Teilen Hydrochinonmonomethylether, 1,5 Teile Triphenylphosphit und 1,5 Teile Hypophosphoriger Säure zugesetzt. Es werden 130 Teile Wasser abgetrennt bevor das Schleppmittel durch Vakuumdestillation entfernt wird. Das Produkt wird über K300-Filter gereinigt. Die Säurezahl beträgt 30 mg KOH/g. Die Viskosität des fast farblosen Produktes (Jodfarbzahl 0-1) liegt bei 380 mPas.

Beispiel 8 (ca. 5fach je Glycerinmolekül ethoxyliertes Glycerin Triacrylat)

940 Teile ca. 5fach ethoxyliertes Glycerin (Lupranol® VP9209, BASF Schwarzheide GmbH) wurde mit 670 Teilen Acrylsäure und 6 Teilen Schwefelsäure in 500 Teilen Methylcyclohexan verestert. Als Hilfsstoffe wurden 3 Teilen Hydrochinonmonomethylether, 1,5 Teil Triphenylphosphit und 1,5 Teil Hypophosphoriger Säure zugesetzt. Es werden 44 Teile Wasser abgetrennt bevor das Schleppmittel durch Vakuumdestillation entfernt wird. Das Produkt wird über K300-Filter gereinigt. Die Säurezahl beträgt 29 mg KOH/g.

Beispiel 9 (ca. 9fach je Glycerinmolekül ethoxyliertes Glycerin Triacrylat)

704 Teile ca. 9fach ethoxyliertes Glycerin (Lutron® HF1, BASF AG) wurde mit 363 Teilen Acrylsäure und 5 Teilen Schwefelsäure in 356 Teilen Methylcyclohexan verestert. Als Hilfsstoffe wurden 3 Teilen Hydrochinonmonomethylether, 1,5 Teil Triphenylphosphit und 1,5 Teil Hypophosphoriger Säure zugesetzt. Es wurden 76 Teile Wasser abgetrennt bevor das Schleppmittel durch Vakuumdestillation entfernt wurde. Das Produkt wurde über K300-Filter gereinigt. Die Säurezahl betrug 71 mg KOH/g und wurde durch Zugabe von 8 Teilen Acrylsäure auf eine Säurezahl von 100 mg KOH/g eingestellt. Die Viskosität des fast farblosen Produktes (Jodfarbzahl 0-1) war 113 mPas.

Beispiel 10 (ca. 5fach je Molekül Pentaerythritol ethoxyliertes Pentaerytrithol Tetraacrylat)

382 Teile ca. 5fach ethoxyliertes Pentaerytrithols werden mit 348 Teilen Acrylsäure und 5 Teilen Schwefelsäure in 180 Teilen Methylcyclohexan verestert. Als Hilfsstoffe werden 3 Teilen Hydrochinonmonomethylether, 1,5 Teil Triphenylphosphit und 1,5 Teil Hypophosphoriger Säure zugesetzt. Es werden 72 Teile Wasser abgetrennt bevor das Schleppmittel durch Vakuumdestillation entfernt wird. Das Produkt wird über K300-Filter gereinigt. Die Säurezahl liegt bei 35 mg KOH/g. Die Viskosität des dunkel gefärbten Produktes (Jodfarbzahl nicht bestimmbar) liegt bei 280 mPas.

Beispiel 11 (ca. 13fach je Molekül ethoxyliertes Dipentaerytrithol)

545 Teile ca. 13fach ethoxyliertes Dipentaerytrithols (DPP 130 der Firma Perstorp AB) werden mit 585 Teilen Acrylsäure und 5 Teilen Schwefelsäure in 400 Teilen Methylcyclohexan verestert. Als Hilfsstoffe werden 3 Teile Hydrochinonmonomethylether, 1,5 Teile Triphenylphosphit und 1,5 Teil Hypophosphoriger Säure zugesetzt. Es werden 130 Teile Wasser abgetrennt bevor das Schleppmittel durch Vakuumdestillation entfernt wird. Das Produkt wird über K300-Filter gereinigt. Die Säurezahl liegt bei 45 mg KOH/g. Die Viskosität des leicht gefärbten Produktes (Jodfarbzahl 1-2)

liegt bei 1600 mPas.

Beispiel 12 (ca. 4 fach je Molekül ethoxyliertes Sorbitol Acrylat)

490 Teile ca. 4fach ethoxyliertes Sorbitol werden mit 444 Teilen Acrylsäure und 5 Teilen Schwefelsäure in 448 Teilen Toluol verestert. Als Hilfsstoffe werden 3 Teilen Hydrochinonmonomethylether, 1,5 Teil Triphenylphosphit und 1,5 Teil Hypophosphoriger Säure zugesetzt. Es werden 96 Teile Wasser abgetrennt bevor das Schleppmittel durch Vakuumdestillation entfernt wird. Das Produkt wird über K300-Filter gereinigt. Die Säurezahl liegt bei 45 mg KOH/g. Die Viskosität des dunkel gefärbten Produktes (Jodfarbzahl 3-4) liegt bei 990 mPas.

Beispiel 13 (ca. 6 fach je Molekül ethoxyliertes Sorbitol Acrylat)

601 Teile ca. 6fach ethoxyliertes Sorbitol werden mit 444 Teilen Acrylsäure und 5 Teilen Schwefelsäure in 448 Teilen Cyclohexan verestert. Als Hilfsstoffe werden 3 Teilen Hydrochinonmonomethylether, 1,5 Teil Triphenylphosphit und 1,5 Teil Hypophosphoriger Säure zugesetzt. Es werden 96 Teile Wasser abgetrennt bevor das Schleppmittel durch Vakuumdestillation entfernt wird. Das Produkt wird über K300-Filter gereinigt. Die Säurezahl liegt bei 45 mg KOH/g. Die Viskosität des dunkel gefärbten Produktes (Jodfarbzahl 2-3) liegt bei 700 mPas.

Beispiel 14 (ca. 8 fach je Molekül ethoxyliertes Sorbitol Acrylat)

689 Teile ca. 8fach ethoxyliertes Sorbitol werden mit 444 Teilen Acrylsäure und 5 Teilen Schwefelsäure in 448 Teilen Toluol verestert. Als Hilfsstoffe werden 3 Teilen Hydrochinonmonomethylether, 1,5 Teil Triphenylphosphit und 1,5 Teil Hypophosphoriger Säure zugesetzt. Es werden 100 Teile Wasser abgetrennt bevor das Schleppmittel durch Vakuumdestillation entfernt wird. Das Produkt wird über K300-Filter gereinigt. Die Säurezahl liegt bei 45 mg KOH/g. Die Viskosität des dunkel gefärbten Produktes (Jodfarbzahl 5) liegt bei 700 mPas.

Beispiel 15 (ca. 10 fach je Molekül ethoxyliertes Sorbitol Acrylat)

788 Teile ca. 8fach ethoxyliertes Sorbitol werden mit 444 Teilen Acrylsäure und 5 Teilen Schwefelsäure in 448 Teilen Toluol verestert. Als Hilfsstoffe werden 3 Teilen Hydrochinonmonomethylether, 1,5 Teil Triphenylphosphit und 1,5 Teil Hypophosphoriger Säure zugesetzt. Es werden 106 Teile Wasser abgetrennt bevor das Schleppmittel durch Vakuumdestillation entfernt wird. Das Produkt wird über K300-Filter gereinigt. Die Säurezahl liegt bei 45 mg KOH/g. Die Viskosität des dunkel gefärbten Produktes (Jodfarbzahl 10-15) liegt bei 500 mPas.

Beispiel 16 (ca. 13 fach je Molekül ethoxyliertes Sorbitol Hexaacrylat)

625 Teile ca. 13fach ethoxyliertes Sorbitol wurde mit 518 Teilen Acrylsäure und 5 Teilen Schwefelsäure in 381 Teilen Methylcyclohexan verestert. Als Hilfsstoffe wurden 3 Teilen Hydrochinonmonomethylether, 1,5 Teil Triphenylphosphit und 1,5 Teil Hypophosphoriger Säure zugesetzt. Es wurden 116 Teile Wasser abgetrennt bevor das Schleppmittel durch Vakuumdestillation entfernt wurde. Das Produkt wird über K300-Filter gereinigt. Die Säurezahl betrug 78 mg KOH/g. Die Viskosität des dunkel gefärbten Produktes (Jodfarbzahl nicht bestimmbar) war 406 mPas.

Herstellung von Hydrogelen

Zur Bestimmung der Güte der Oberflächenvernetzung kann das getrocknete Hydrogel mit folgenden Testmethoden untersucht werden.

Testmethoden

a) Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0,2000 ± 0,0050 g getrocknetes Hydrogel (Kornfraktion 106 - 850 μm) in einem 60 x 85 mm großen Teebeutel eingewogen, der anschließend verschweißt wird. Der Teebeutel wird für 30 Minuten in einen Überschuss von 0,9 Gew.%igen Kochsalzlösung gegeben (mindestens 0,83 1 Kochsalz-Lösung / 1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

b) Absorption unter Druck (AUL Absorbency Under Load) (0,7 psi)

Die Messzelle zur Bestimmung der AUL 0,7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1345 g. Zur Durchführung der Bestimmung der AUL 0,7 psi wird das Gewicht des leeren Plexiglas-Zylinders und der Plastikplatte ermittelt und als Wo notiert. Dann werden 0,900 ± 0,005 g Hydrogel-formendes Polymer (Korngrößenverteilung 150 - 800 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0,9 Gew.%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 0,7 psi [g/g]} = [W_b - W_a] \,/\, [W_a - W_0]$$

Der AUL 0,5psi wird analog mit geringerem Druck gemessen.

c) Die Bestimmung des nach 16 h extrahierbare Anteils (Extract. 16h) erfolgte analog, wie in EP-A1 811 636, S. 13, Z. 1 bis Z. 19 beschrieben.

Beispiel 18-27:

Herstellung des Grundpolymers

In einem Laborkneter (Werner und Pfleiderer LUK 8.0 K2) wurden 6 kg einer 40 Gew.%igen wäßrigen Acrylsäurelösting vorgelegt, die zu 77 mol% mit Natriumhydroxid neutralisiert worden war.

Als Vernetzer wurden die in Tabelle 1 angegebenen Typen in den dort angegebenen Mengen jeweils bezogen auf auf eingesetzte Acrylsäure zugegeben. Danach wurden als Polymerisationsstarter 0,28 Gew.% Natriumpersulfat und 0,0056 Gew.% Ascorbinsäure -jeweils bezogen auf eingesetztes Acrylsäuremonomer- zugegeben.

Die Reaktion startete, und die Temperatur des Knetermantels wurde so nachgeregelt, daß die Reaktionswärme nicht über den Mantel abgeführt wurde. Dadurch kommt es zu einer fast adiabatischen Aufheizung der Reaktionsmischung wobie die Polymerisation unter Rühren stattfindet. Am Endpunkt der Reaktion wird die Temperatur noch ca. eine Stunde beibehalten. Danach konnte jeweils ein feinkrümmeliges Gel entleert werden.

Das Gel wurde für 3h bei 160 C im Umluftschrank getrocknet, mit einem Laborwalzenstuhl gemahlen, und bei 100 - 850 Mikrometer abgesiebt. Dies ist das normale Grundpolymer der Tabelle 1.

Alternativ wurde das Gel zunächst 6h bei 90 C im versiegelten Plastikbeutel getempert, und erst danach wurde für 3h bei 160 C im Umluftschrank getrocknet, mit einem Laborwalzenstuhl gemahlen, und schließlich bei 100 - 850 Mikrometer abgesiebt. Dies ist das hydrolysierte Grundpolymer der Tabelle 1.

Nachvernetzung:

Das trockene normale Grundpolymerpulver wurde mit einer Lösung aus 0,06 Gew. % Ethylenglykoldiglycidylether (Firma Nagase, Japan), 3,43 Gew.% Wasser und 1,47 Gew.% Propandiol-1,2 -jeweils bezogen auf eingesetztes Polymer- unter Rühren homogen besprüht. Das feuchte Pulver wurde dann im Trockenschrank bei 150 C für 60 min getempert. Danach wurde nochmals bei 850 Mikrometer gesiebt um Agglomerate zu entfernen. Die Eigenschaften dieses nachvernetzten Polymers wurden bestimmt und sind in Tabelle 1 aufgeführt.

Tabelle 1

| Beispiel | Vernetzer | Einsatzmenge | Löslichkeit | **Grundpolymer** | | Nachvernetztes Polymer | | |
|---|---|---|---|---|---|---|---|---|
| | | | | CRC | Extract. 16h | CRC | AUL 0.7 psi | Extract. 16h |
| 18 | Produkt aus Beispiel 1 | 0,44% | Klar | 36,2 | 9,9% | 27,9 | 25,8 | 7,2% |
| 19 | Produkt aus Beispiel 9 | 0,50% | Klar | 31,0 | 7,5% | 29,2 | 25,8 | 7,2% |
| 20 | Produkt aus Beispiel 4 | 0,50% | | 35,4 | 9,4% | 29,9 | 25,8 | 6,1% |
| 21 | Produkt aus Beispiel 5 | 0,50% | | 38,1 | 13,8% | 32,8 | 24,7 | 11,6% |
| 22 | Produkt aus Beispiel 6 | 0,50% | | 35,3 | 10,0% | 31,2 | 26,4 | 8,0% |

Die Löslichkeit des Vernetzers wird durch Zugabe des Vernetzers zur Reaktionsmischung bei Raumtemperatur bestimmt. Die Beurteilung erfolgt visuell.

Folgende Skala wird verwendet
Klar = vollständige klare Lösung
Trüb = mit bloßem Auge beobachtbare deutliche Trübung in der Lösung
Tropfen = Aufschwimmen des Vernetzers in Tropfenform
Die anderen erfindungsgemäßen Produkte können analog umgesetzt werden.

## Patentansprüche

1. Vernetztes Hydrogel, enthaltend mindestens ein hydrophiles Monomer M in einpolymerisierter Form, vernetzt mit einem Ester F eines Polyalkohols A mit mindestens einer ethylenisch ungesättigten Carbonsäure B, wobei es sich bei dem Polyalkohol A um ein pro Glycerin drei bis vierfach ethoxyliertes Glycerin oder um ein pro Hydroxygruppe vierfach ethoxyliertes Trimethylolpropan oder Pentaerythrit handelt.

2. Vernetztes Hydrogel gemäß Anspruch 1, **dadurch gekennzeichnet, daß** das hydrophile Monomer M ausgewählt ist aus der Gruppe bestehend aus Acrylsäure, Methacrylsäure, Ethacrylsäure, α-Chloracrylsäure, Crotonsäure, Maleinsäure, Maleinsäureanhydrid, Vinylsulfonsäure, Vinylphosphonsäure, Maleinsäure einschließlich deren Anhydrid, Fumarsäure, Itaconsäure, Citraconsäure, Mesaconsäure, Glutaconsäure, Aconitsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Allylphosphonsäure, Styrolsulfonsäure, 2-Acrylamido-2methylpropansulfonsäure, 2-Acrylamido-2-methylpropanphosphonsäure sowie deren Amide, Hydroxyalkylester und aminogruppen- oder ammoniumgruppenhaltige Ester und Amide.

3. Vernetztes Hydrogel gemäß Anspruch 1, **dadurch gekennzeichnet, daß** das hydrophile Monomer M ausgewählt ist aus der Gruppe bestehend aus Acrylsäure und Methacrylsäure.

4. Vernetztes Hydrogel gemäß einem der vorstehenden Ansprüche, **dadurch gekennzeichnet, daß** die ethylenisch ungesättigte Carbonsäure B ausgewählt ist aus der Gruppe bestehend aus Acrylsäure, Methacrylsäure, Ethacrylsäure, Maleinsäure einschließlich deren Anhydrid, Fumarsäure, Itaconsäure, Citraconsäure, Mesaconsäure, Vinylessigsäure, Allylessigsäure und Crotonsäure.

5. Vernetztes Hydrogel gemäß einem der Ansprüche 1 bis 3, **dadurch gekennzeichnet, daß** die ethylenisch ungesättigte Carbonsäure B ausgewählt ist aus der Gruppe bestehend aus Acrylsäure und Methacrylsäure.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9321237 A
- US 4187383 A
- WO 200114438 A
- WO 200110920 A
- EP A A
- EP 279303 A
- DE 19941136 A
- DE 3843843 A
- DE 3843854 A
- DE 19937911 A
- DE 19929258 A
- EP 331845 A
- EP 554651 A
- DE 3843854 A1
- US 4286082 A
- DE 2706135 C
- US 4340706 A
- DE 3713601 C
- DE 2840010 C
- DE 4344548 A
- DE 020780 A4
- DE 4015085 A
- DE 3917846 A
- DE 3807289 A
- DE 3533337 A
- DE 3503458 A
- DE 4244548 A
- DE 4219607 A
- DE 4021847 A
- DE 3831261 A
- DE 3511086 A
- DE 3118172 A
- DE 3028043 A
- DE 4418881 A
- EP 0801483 A
- EP 0455985 A
- EP 0467073 A
- EP 0312952 A
- EP 0205874 A
- EP 0499774 A
- DE 2612846 A
- DE 4020780 A
- EP 0205674 A
- US 5145906 A
- EP 0530438 A
- EP 0670073 A
- US 4057521 A
- US 4062817 A
- US 4525527 A
- US 4295987 A
- US 5011892 A
- US 4076663 A
- US 4931497 A
- WO 0138402 A
- DE 19854575
- US 5041496 A
- EP 0343427 A
- DE 1301566 C
- WO 9957355 A1
- EP 1023883 A2
- DE 19604601 A1
- EP 0316518 A
- EP 0202127 A
- WO 0065084 A
- WO 0065348 A
- WO 0035502 A
- DE 19737434
- WO 988439 A
- WO 9524173 A
- WO 9111977 A
- EP 389023 A
- WO 9425077 A
- WO 9701317 A
- WO 9918905 A
- EP 834297 A
- US 5762644 A
- US 5895381 A
- WO 9857609 A
- WO 2000065083 A
- WO 2000069485 A
- WO 2000069484 A
- WO 2000069481 A
- US 6123693 A
- EP 1104666 A
- WO 2001024755 A
- WO 2001000115 A
- EP 105373 A
- WO 2001041692 A
- EP 1074233 A
- WO 9848753 A
- WO 9841179 A
- WO 9709022 A
- WO 9846182 A
- WO 9846181 A
- WO 2001043679 A
- WO 2001043680 A
- WO 2000061052 A
- EP 1108408 A

- WO 2001033962 A
- DE 200020662
- WO 2001001910 A
- WO 2001001908 A
- WO 2001001909 A
- WO 2001001906 A
- WO 2001001905 A
- WO 200124729 A
- EP 311344 A
- EP 850623 A
- WO 9526207 A
- EP 894502 A
- EP 850616 A
- WO 9822063 A
- WO 9749365 A
- EP 903134 A
- EP 887060 A
- EP 887059 A
- EP 887058 A
- EP 887057 A
- EP 887056 A
- EP 931530 A
- WO 9925284 A
- WO 9322998 A
- WO 9526209 A
- WO 9820916 A
- EP 306262 A
- WO 9945973 A
- WO 9828478 A
- EP 0615736 A1
- WO 200036216 A1
- US 3556932 A
- WO 9111162 A
- US 3224926 A
- US 3440135 A
- US 3932209 A
- US 4035147 A
- US 4822453 A
- US 4888093 A
- US 4898642 A
- US 5137537 A
- EP A1 A
- EP 811636 A

**In der Beschreibung aufgeführte Nicht-Patentliteratur**

- Die Methode ist im Analytiker Taschenbuch. vol. 4
- **Houben-Weyl.** Methoden der Organischen Chemie. Thieme Verlag Stuttgart, 1979, vol. 6/1a, 373-385
- **R. Billet.** Verdampfertechnik. HTB-Verlag, 1965, 53
- **F.L. Buchholz ; A.T. Graham.** Modern Superabsorbent Polymer Technology. Wiley-VCH, 1998
- **von Robert F. Gould.** Kontaktwinkel, Benetzbarkeit und Adhäsion. American Chemical Society, 1964